# EUROPEAN PATENT SPECIFICATION

(11) **EP 0 533 840 B1**
(45) Date of publication and mention of the grant of the patent: **11.12.1996**
(21) Application number: 91912884.3
(22) Date of filing: 07.06.1991
(51) Int. Cl.: A61K 31/415

(54) **TREATMENT OF CHRONIC RENAL FAILURE WITH IMIDAZOLE ANGIOTENSIN-II RECEPTOR ANTAGONISTS**
BEHANDLUNG DES CHRONISCHEN NIERENVERSAGENS MIT IMIDAZOL-ANGIOTENSIN-II-REZEPTORANTAGONISTEN
TRAITEMENT DE L'INSUFFISANCE RENALE CHRONIQUE A L'AIDE D'IMIDAZOLES ANTAGONISTES DES RECEPTEURS D'ANGIOTENSINE-II

(30) Priority: 22.06.1990 US 542351
(43) Date of publication of application: 31.03.1993
(73) Proprietor: E.I. DU PONT DE NEMOURS AND COMPANY, Wilmington Delaware 19898 (US)
(72) Inventor: CARINI, David, John, Wilmington, DE 19803 (US); DUNCIA, John, Jonas, Vytautas, Newark, DE 19711 (US); WONG, Pancras, Chor-Bun, Wilmington, DE 19808 (US)
(74) Representative: von Kreisler, Alek, Dipl.-Chem.
(86) International application number: US9103906
(87) International publication number: WO9200067

(56) References cited:
- EP-A- 0 323 841
- EP-A- 0 403 159
- EP-A- 0 437 103
- WO-A-91/00277
- Journal of Hypertension Supplement, vol. 7, Suppl. 7, September 1989, CS Current Science, London, GB; L. Raij et al.: "Possible mechanism for the renoprotective effect of antiotensin converting enzyme inhibitors", pages S33- S37, see the abstract
- Brazilian Journal of Medical And Biological Research, vol. 21, no. 2, 1988, R. C. Abdulkader et al.: "Prolonged inhibition of angiotensin II attenuates glycerol-induced acute renal failure", pages 233-239, see the abstract
- Kidney International Supplement 16, vol. 24, December 1983, New York, US; M.O. Magnusson et al.: "Enhancement of recovery in postischemic acute renal failure with captopril", pages S324-S326, see results; discussion
- Transplantation, vol. 36, no. 2, August 1983, US; H. Huland et al.: "The influence of an angiotensin II antagonist saralasin, given before donor nephrectomy, on kidney function after transplantation", pages 139-142, see results; discussion
- American Journal of Hypertension, 4(5):11A-12A (1991)

## Description

### Field of the Invention

This invention relates to the use of particular imidazole compounds for preparing a medicament for treating chronic renal failure mediated by angiotensin-II.

### Background of the Invention

Angiotensin converting enzyme inhibitors may have beneficial effects over other antihypertensive agents in progressive renal disease of various origins including diabetic nephropathy, essential hypertension and other intrinsic renal diseases (See, e.g., Hypertension: Pathophysiology, Diagnosis, and Management, ed. by J. H. Laragh and B. M. Brenner, vol. 1, pp. 1163-1176, Raven Press, Ltd., New York, 1990; Hypertension: Pathophysiology, Diagnosis, and Management, ed. by J. H. Laragh and B. M. Brenner, vol. 2, pp. 1677-1687, Raven Press, Ltd., New York, 1990). For instance, in partially nephrectomized rats, glomerular capillary hypertension in the remnant kidney is associated with progressive proteinuria, focal glomerular sclerosis, and moderate hypertension. Angiotensin converting enzyme inhibitors, which lower systemic arterial pressure and glomerular capillary pressure, limit the progression of glomerular injury.

There are other antihypertensive agents which lower systemic arterial blood pressure to a similar extent, but fail to reduce glomerular capillary pressure. Such agents do not prevent the progression of glomerular injury. It is speculated that in these rats intrarenal generation of angiotensin-II constricts the renal efferent arteriole and causes an increase in glomerular hydraulic pressure. Glomerular hyperfiltration, hyperperfusion and/or hypertension may then initiate and induce glomerular lesions. Thus, blockade of the intrarenal formation of angiotensin-II by angiotensin converting enzyme inhibitors may retard the deterioration of renal failure.

Although the partially nephrectomized rat is associated with moderate hypertension, systemic hypertension does not appear to be necessary for the acceleration of renal disease. In the insulin-treated rat with streptozocin-induced diabetes, systemic arterial pressure is normal but glomerular capillary pressure is high. Similar to the partially nephrectomized rat model, angiotensin converting enzyme inhibitors are beneficial in limiting the glomerular structural lesions, suggesting that glomerular capillary hypertension but not systemic arterial hypertension play a critical role in rats with progressive renal failure.

Nonpeptide angiotensin-II receptor antagonists are believed to be more efficacious than angiotensin converting enzyme inhibitors in treating chronic renal failure because the nonpeptide angiotensin-II receptor antagonists may block the renal effect of angiotensin-II more completely irrespective of the source of angiotensin-II. In contrast, angiotensin converting enzyme inhibitors selectively block kininase II and, thus, may not inhibit totally the local formation of angiotensin-II in the kidney. Other types of peptidyl dipeptidase may also be responsible for the formation of angiotensin-II. (Wong, P. C. and Zimmerman, B. G.: Role of Extrarenal and Intrarenal Converting Enzyme Inhibition in Renal Vasodilator Response to Intravenous Captopril. Life Sci. 27: 1291, 1980; Schmidt M., Giesen-Crouse, E. M., Krieger, J. P., Welsch, C. and Imbs, J. L.: Effect of angiotensin converting enzyme inhibitors on the vasoconstrictor action of angiotensin I on isolated rat kidney. J. Cardiovasc. Pharmacol. 8 (Suppl. 10): S100, 1986)).

In clinical renal artery stenosis, the usefulness of angiotensin converting enzyme inhibitors may be limited by a reversible loss of filtration in the stenotic kidney. It is possible that nonpeptide angiotensin II receptor antagonists may have a similar renal effect in the stenotic kidney.

K. Matsumura, et al., in U.S. Patent 4,207,324 issued June 10, 1980 discloses 1,2-disubstituted-4-haloimidazole-5-acetic acid derivatives of the formula: 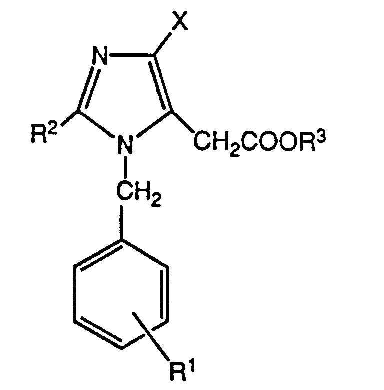 wherein R¹ is hydrogen, nitro or amino; R² is phenyl, furyl or thienyl optionally substituted by halogen, lower alkyl, lower alkoxy or di-lower alkylamino; R³ is hydrogen or lower alkyl and X is halogen; and their physiologically acceptable salts. These compounds have diuretic and hypotensive actions.

Furukawa, et al., in U.S. Patent 4,355,040 issued October 19, 1982 discloses hypotensive imidazole-5-acetic acid derivatives having the formula: 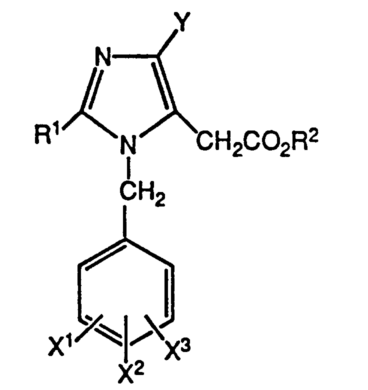 wherein R¹ is lower alkyl, cycloalkyl, or phenyl optionally substituted; X¹, X², and X³ are each hydrogen, halogen, nitro, amino, lower alkyl, lower alkoxy, benzyloxy, or hydroxy; Y is halogen and R² is hydrogen or lower alkyl; and salts thereof.

Furukawa, et al., in U.S. Patent 4,340,598, issued July 20, 1982, discloses hypotensive imidazole derivatives of the formula: 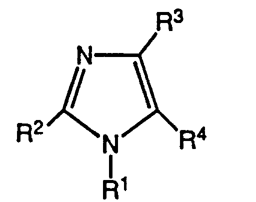 wherein R¹ is lower alkyl or, phenyl C₁₋₂ alkyl optionally substituted with halogen or nitro; R² is lower alkyl, cycloalkyl or phenyl optionally substituted; one of R³ and R⁴ is -(CH₂)ₙCOR⁵ where R⁵ is amino, lower alkoxyl or hydroxyl and n is 0, 1, 2 and the other of R³ and R⁴ is hydrogen or halogen; provided that R¹ is lower alkyl or phenethyl when R³ is hydrogen, n=1 and R⁵ is lower alkoxyl or hydroxyl; and salts thereof.

Furukawa et al., in EP-A-0 103 647 discloses 4-chloro-2-phenylimidazole-5-acetic acid derivatives useful for treating edema and hypertension of the formula: 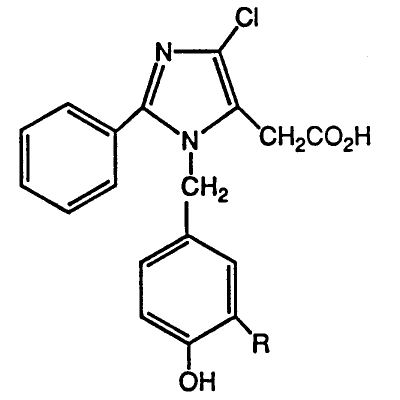 where R represents lower alkyl and salts thereof.

The metabolism and disposition of hypotensive agent 4-chloro-1-(4-methoxy-3-methylbenzyl)-2-phenyl-imidazole-5-acetic acid is discloses by H. Torii in Takeda Kenkyushoho, 41, No 3/4, 180-191 (1982).

Frazee et al., in EP-A-0 125 033 discloses 1-phenyl(alkyl)-2-(alkyl)-thioimidazole derivatives which are inhibitors of dopamine-β-hydroxylase and are useful as antihypertensives, diuretics and cardiotonics. Further EP-A-0 403 159 and EP-A-0 437 103 (both documents being relevant under Art. 54(3) EPC) are disclosing angiotensin-II receptor antagonists useful for the treatment of renal failure, which antagonists are having the formula 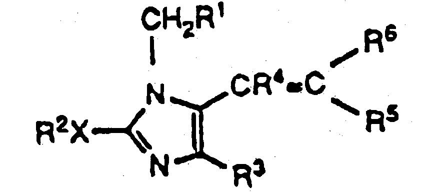 in which:
R¹ is phenyl, biphenyl, naphthyl, or adamantylmethyl, which are unsubstituted or substituted by one to three substituents selected from Cl, Br, F, I, C₁-C₄alkyl, nitro, CO₂R⁷, tetrazol-5-yl, C₁-C₄alkoxy, hydroxy, SC₁-C₄alkyl, SO₂NHR⁷, NHSO₂R⁷, SO₃H, CONR⁷R⁷, CN, SO₂C₁-C₄alkyl, or CₙF₂ₙ₊₁, wherein n is 1-3;
R² is C₂-C₁₀alkyl, C₃-C₁₀alkenyl, C₃-C₁₀alkynyl, C₃-C₆cycloalkyl, or (CH₂)₀₋₈phenyl unsubstituted or substituted by one to three substituents selected from C₁-C₄alkyl, nitro, Cl, Br, F, I, hydroxy, C₁-C₄alkoxy, NR⁷R⁷, CO₂R⁷, CN, or CONR⁷R⁷;
X is a single bond, S, or O;
R³ is hydrogen, Cl, Br, F, I, CHO, hydroxymethyl, COOR⁷, CONR⁷R⁷, NO₂, or CₙF₂ₙ₊₁, wherein n is 1-3;
R⁴ and R⁵ are independently hydrogen, C₁-C₆alkyl, thienyl-Y-, pyrazolyl-Y-, imidazolyl-Y-, thiazolyl-Y-, furyl-Y-, pyrrolyl-Y-, triazolyl-Y-, oxazolyl-Y-, isoxazolyl-Y-, pyridyl-Y-, or tetrazolyl-Y-, except that R⁴ and R⁵ are not both selected from hydrogen and C₁-C₆alkyl and each heterocyclic ring is unsubstituted or substituted by C₁-C₆alkyl, C₁-C₆ alkoxy, Cl, Br, F, I, NR⁷R⁷, CO₂R⁷, SO₂NHR⁷, SO₃H, or CONR⁷R⁷;
Y is a single bond, S, O, or C₁-C₆alkyl which is straight or branched or optionally substituted by phenyl or benzyl, wherein each of the aryl groups is unsubstituted or substituted by halo, NO₂, CF₃, C₁-C₄alkyl, C₁-C₄alkoxy, CN, or CO₂R⁷;
R⁵ is -Z-COOR⁸ or -Z-CONR⁷R⁷;
Z is a single bond, vinyl, -CH₂-O-CH₂-, methylene optionally substituted by C₁-C₄alkyl, one or two benzyl groups, thienylmethyl, or furylmethyl, or -C(O)NHCHR⁹-, wherein R⁹ is H, C₁-C₄alkyl, phenyl, benzyl, thienylmethyl, or furylmethyl;
each R⁷ independently is hydrogen, C₁-C₄alkyl, or (CH₂)ₘphenyl, wherein m is 0-4; and
R⁸ is hydrogen, C₁-C₆alkyl, or 2-di(C₁-C₄alkyl)amino-2-oxoethyl; or a pharmaceutically acceptable salt thereof
and are having the formula 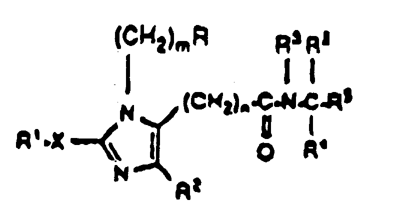 in which:
R is adamantyl, or naphthyl, biphenyl, or phenyl, with each aryl group being unsubstituted or substituted by one to three substituents selected from halo, C₁₋₆alkyl, C₁₋₆alkoxy, OH, CN, CO₂R³, tetrazol-5-yl, SO₃H, SO₂NHR³, NO₂, W, SC₁₋₆alkyl, SO₂C₁₋₆alkyl,NHSO₂R³, PO(OR³)₂, CONR³R³, NR³R³, NR³COH, NR³COC₁₋₆alkyl, NR³CON(R³)₂, NR³COW, or SO₂W;
R¹ is C₂₋₁₀alkyl, C₃₋₁₀alkenyl, (CH₂)₀₋₈C₃₋₆cycloalkyl, or (CH₂)₀₋₈phenyl unsubstituted or substituted by one to three substituents selected from C₁₋₆alkyl, C₁₋₆alkoxy, halo, OH, NO₂, NR³R³, W, CO₂R³, CN, CONR³R³, NR³COH, tetrazol-5-yl, NR³COC₁₋₆alkyl, NR³COW, SC₁₋₆alkyl, SO₂W, or SO₂C₁₋₆alkyl;
X is a single bond, S, NR³, or O;
m is 0-4;
R² is H, C₁₋₆alkyl, halo, W, CHO, CH₂OH, CO₂R³, CONR³R³, NO₂, CN, NR³R³, or phenyl;
each R³ independently is H or C₁₋₆alkyl;
R⁴ is H, C₁₋₈alkyl, thienyl-Y-, furyl-Y-, pyrazolyl-Y, imidazolyl-Y-, thiazolyl-Y-, pyridyl-Y-, tetrazolyl-Y-, pyrrolyl-Y-, triazolyl-Y-, oxazolyl-Y-, isoxazolyl-Y-, or phenyl-Y-, with each aryl or heteroaryl group being unsubstituted or substituted by C₁₋₈alkyl, C₁₋₈alkoxy, halo, NR³R³, CO₂R³, OH, NO₂, SO₂NHR³, SO₃H, CONR³R³, W, SO₂W, SC₁₋₆alkyl, SO₂C₁₋₆alkyl, NR³C(O)W, or NR³C(O)C₁₋₆alkyl;
R⁵ is CO₂R³, CONR³R³ or tetrazol-5-yl;
W is C₁F_{2q+1}, wherein q is 1-4;
Y is a single bond or C₁₋₆alkyl which is straight or branched; and
n is 0-5; or a pharmaceutically acceptable salt thereof.

EP-A-0 146 228 filed October 16,1984 by S. S. L. Parhi discloses a process for the preparation of 1-substituted-5-hydroxymethyl-2-mercaptoimidazoles.

A number of references disclose 1-benzyl-imidazoles such as U.S. Patent 4,448,781 to Cross and Dickinson (issued May 15, 1984); U.S. Patent 4,226,878 to Ilzuka et al. (issued October 7, 1980); U.S. Patent 3,772,315 to Regel et al. (issued November 13, 1973); and U.S. Patent 4,379,927 to Vorbrüggen et al. (issued April 12, 1983).

Pals et al., Circulation Research, 29, 673 (1971) describe the introduction of a sarcosin residue in position 1 and alanine in position 8 of the endogenous vasoconstrictor hormone AII to yield an (octa)peptide that blocks the effects of AII on the blood pressure of pithed rats. This analog, [Sar¹, Ala⁸] AII, initially called "P-113" and subsequently "Saralasin", was found to be one of the most potent competitive antagonists of the actions of AII, although, like most of the so-called peptide-AII-antagonists, it also possesses agonistic actions of its own. Saralasin has been demonstrated to lower arterial pressure in mammals and man when the (elevated) pressure is dependent on circulating AII (Pals et al., Circulation Research, 29, 673 (1971); Streeten and Anderson, Handbook of Hypertension, Vol. 5, Clinical Pharmacology of Antihypertensive Drugs, A. E. Doyle (Editor), Elsevier Science Publishers B. V., p. 246 (1984)). However, due to its agonistic character, saralasin generally elicits pressor effects when the pressure is not sustained by AII. Being a peptide, the pharmacological effects to saralasin are relatively short-lasting and are only manifest after parenteral administration, oral doses being ineffective. Although the therapeutic uses of peptide AII-blockers, like saralasin, are severely limited due to their oral ineffectiveness and short duration of action, their major utility is as a pharmaceutical standard.

### Summary of the Invention

According to the present invention there is provided the use of a compound having the formula (I): 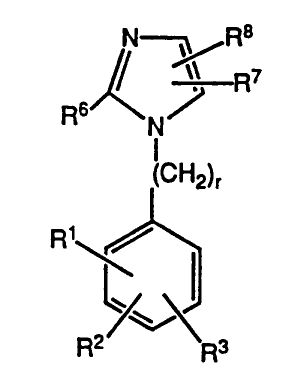 wherein
- R¹: is 4-CO₂H; 4-CO₂R⁹; 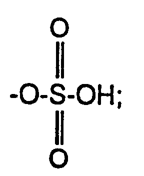 -SO₃H; -C(CF₃)₂OH; 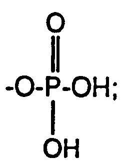 -PO₃H₂; 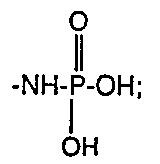 4-NHSO₂CH₃; -4-NHSO₂CF₃; -CONHOR¹²; -SO₂NH₂; 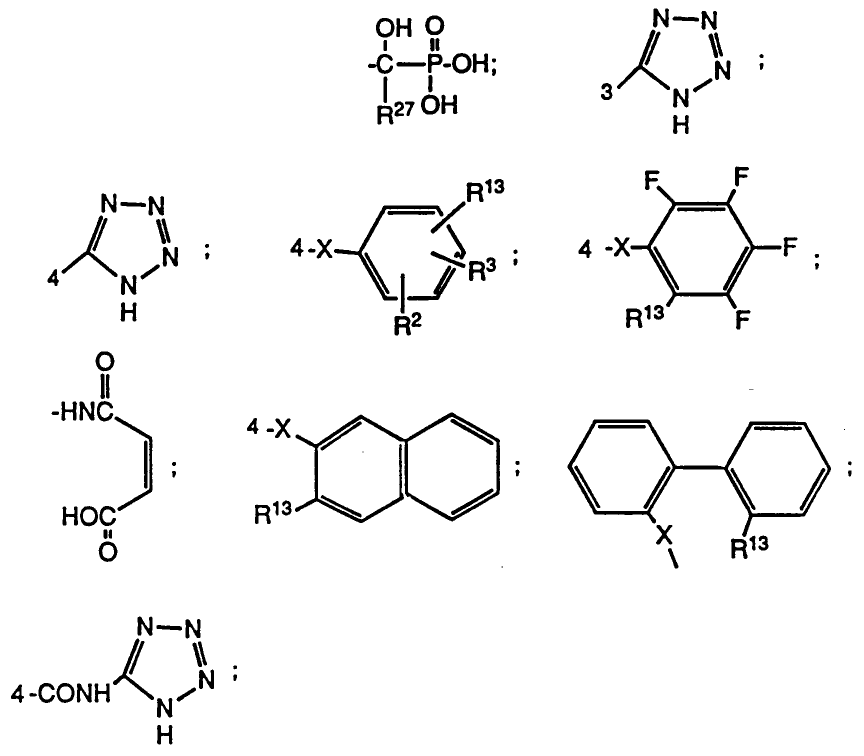 4-CONHNHSO₂CF₃; 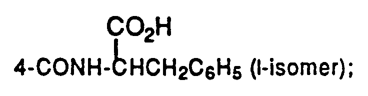 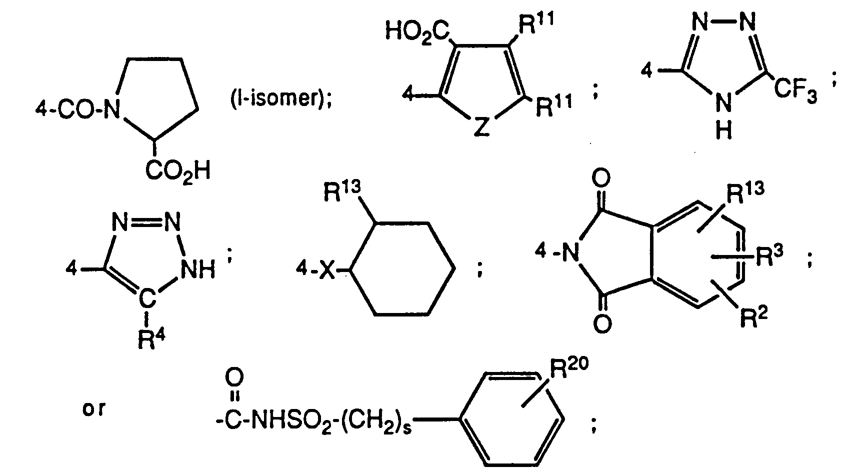
- R²: is H; Cl; Br; I; F; NO₂; CN; alkyl of 1 to 4 carbon atoms; acyloxy of 1 to 4 carbon atoms; alkoxy of 1 to 4 carbon atoms; CO₂H; CO₂R⁹; NHSO₂CH₃; NHSO₂CF₃; CONHOR¹²; SO₂NH₂; 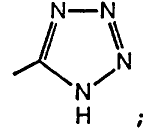 aryl; or furyl;
- R³: is H; Cl, Br, I or F; alkyl of 1 to 4 carbon atoms or alkoxy of 1 to 4 carbon atoms;
- R⁴: is CN, NO₂ or CO₂R¹¹;
- R⁵: is H, alkyl of 1 to 6 carbon atoms, cycloalkyl of 3 to 6 carbon atoms, alkenyl or alkynyl of 2 to 4 carbon atoms;
- R⁶: is alkyl of 2 to 10 carbon atoms, alkenyl or alkynyl of 3 to 10 carbon atoms or the same groups substituted with F or CO₂R¹⁴; cycloalkyl of 3 to 8 carbon atoms, cycloalkylalkyl of 4 to 10 carbon atoms; cycloalkylalkenyl or cycloalkylalkynyl of 5 to 10 carbon atoms; (CH₂)ₛZ(CH₂)ₘR⁵ optionally substituted with F or CO₂R¹⁴; benzyl or benzyl substituted on the phenyl ring with 1 or 2 halogens, alkoxy of 1 to 4 carbon atoms, alkyl of 1 to 4 carbon atoms or nitro;
- R⁷: is H, F, Cl, Br, I, NO₂, CᵥF₂ᵥ₊₁, where v = 1-6; C₆F₅; CN; 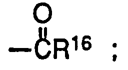 straight or branched alkyl of 1 to 6 carbon atoms; phenyl or phenylalkyl, where alkyl is 1 to 3 carbon atoms; or substituted phenyl or substituted phenylalkyl, where alkyl is 1 to 3 carbon atoms, substituted with one or two substituents selected from alkyl of 1 to 4 carbon atoms, F, Cl, Br, OH, OCH₃, CF₃, and COOR, where R is H, alkyl of 1 to 4 carbon atoms, or phenyl; vinyl; alkynyl of 2-10 carbon atoms; phenylalkynyl where the alkynyl portion is 2-6 carbon atoms; heteroaryl selected from 2- and 3-thienyl, 2- and 3-furyl, 2-, 3-, and 4-pyridyl, 2-pyrazinyl, 2-, 4-, and 5-pyrimidinyl, 3- and 4-pyridazinyl, 2-, 4- and 5-thiazolyl, 2-, 4-, and 5-selenazolyl, 2-, 4-, and 5-oxazolyl; 2- or 3-pyrrolyl, 3-, 4- or 5-pyrazolyl, and 2-, 4- or 5-imidazolyl; o-, m- or p-biphenylyl; o-, m- or p-phenoxyphenyl; substituted phenylalkynyl, heteroaryl, biphenyl or phenoxyphenyl as defined above substituted on ring carbon with 1 or 2 substituents selected from halogen, alkoxy of 1-5 carbon atoms, alkyl of 1-5 carbon atoms, -NO₂, -CN, -CF₃, -COR¹⁶, -CH₂OR¹⁷, -NHCOR¹⁷, CONR¹⁸R¹⁹, S(O)ᵣR¹⁷, and SO₂NR¹⁸R¹⁹; pyrrolyl, pyrazolyl or imidazolyl as defined above substituted on ring nitrogen with alkyl of 1-5 carbon atoms or benzyl; or substituted alkyl, alkenyl, or alkynyl of 1 to 10 carbon atoms substituted with a substituted or unsubstituted heteroaryl, biphenylyl or phenoxyphenyl group as defined above; any of the foregoing polycyclic aryl groups substituted with 1 or 2 substituents selected from halogen, alkoxy of 1-5 carbon atoms, alkyl of 1-5 carbon atoms, -NO₂, -CN, -CF₃, -COR¹⁶, -CH₂OR¹⁷, -NHCOR¹⁷, CONR¹⁸R¹⁹, S(O)ᵣR¹⁷, and SO₂NR¹⁸R¹⁹; the anhydride of 4,5-dicarboxyl-1- or 2-naphthyl; or substituted alkyl of 1 to 10 carbon atoms, alkenyl or alkynyl of 2 to 10 carbon atoms substituted with a substituted or unsubstituted polycyclic aryl group as defined above;
- R⁸: is H, CN, alkyl of 1 to 10 carbon atoms, alkenyl of 3 to 10 carbon atoms, or the same groups substituted with F; phenylalkenyl wherein the aliphatic portion is 2 to 6 carbon atoms; -(CH₂)ₘ-imidazol-1-yl; -(CH₂)ₘ-1,2,3-triazolyl optionally substituted with one or two groups selected from CO₂CH₃ or alkyl of 1 to 4 carbon atoms; -(CH₂)ₛ-tetrazolyl; 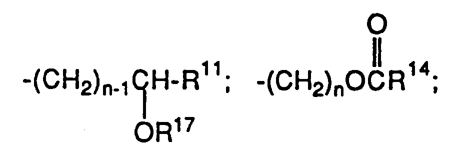 -(CH₂)ₙSR¹⁵; 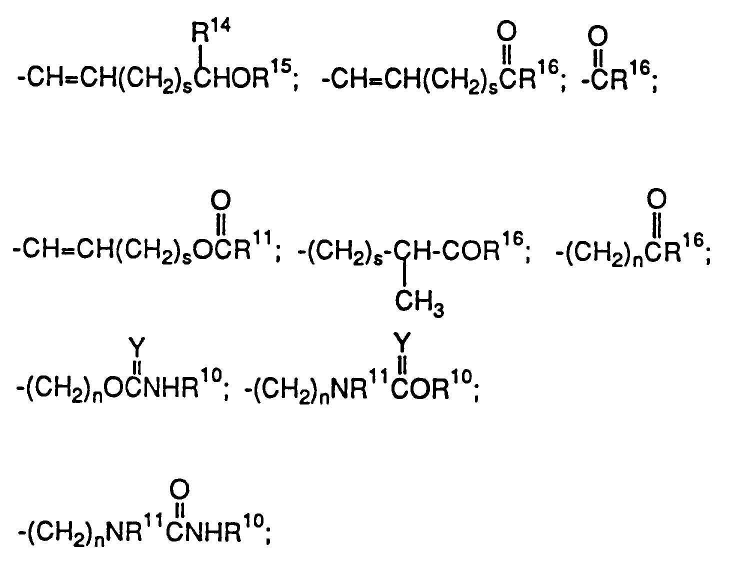 -(CH₂)ₙNR¹¹SO₂R¹⁰; 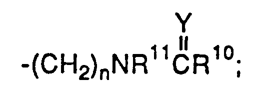 -(CH₂)ₘF; -(CH₂)ₘONO₂; -CH₂N_{3;} -(CH₂)ₘNO₂; -CH=N-NR¹¹R¹⁷; 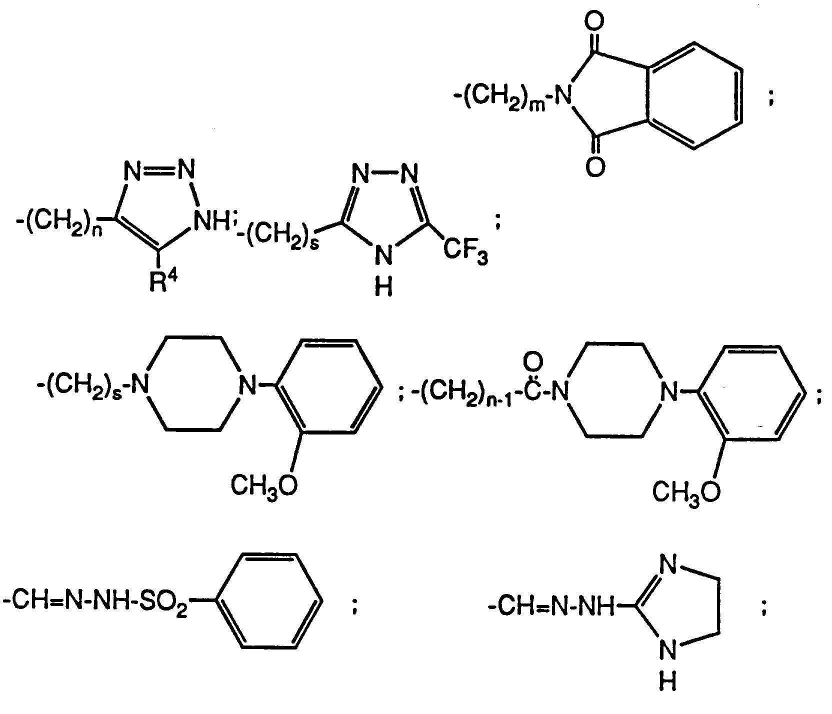
- R⁹: is 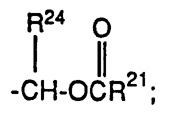
- R¹⁰: is alkyl of 1 to 6 carbon atoms or perfluoroalkyl of 1 to 6 carbon atoms, 1-adamantyl, 1-naphthyl, 1-(1-naphthyl)ethyl, or (CH₂)ₚC₆H₅;
- R¹¹: is H, alkyl of 1 to 6 carbon atoms, cycloalkyl of 3 to 6 carbon atoms, phenyl or benzyl;
- R¹²: is H, methyl or benzyl;
- R¹³: is -CO₂H; -CO₂R⁹; -CH₂CO₂H, -CH₂CO₂R⁹; 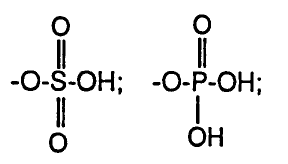 -SO₃H; 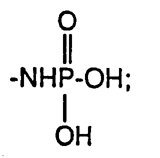 -PO₃H₂; -C(CF₃)₂OH; -NHSO₂CH₃; -NHSO₂CF₃; -NHCOCF₃; -CONHOR¹²; -SO₂NH₂; 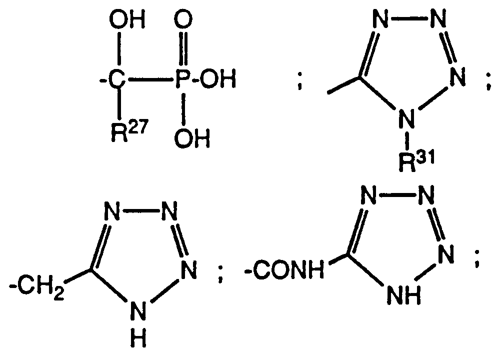 -CONHNHSO₂CF₃ ; 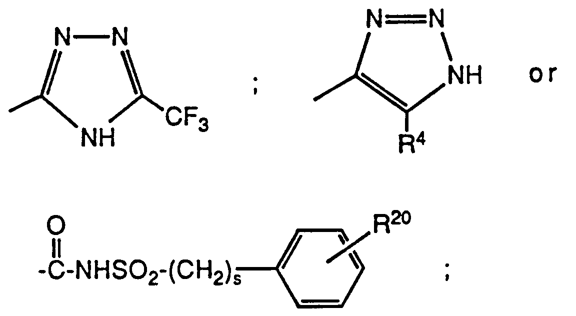
- R¹⁴: is H, alkyl or perfluoroalkyl of 1 to 8 carbon atoms, cycloalkyl of 3 to 6 carbon atoms, phenyl or benzyl;
- R¹⁵: is H, alkyl of 1 to 6 carbon atoms, cycloalkyl of 3 to 6 carbon atoms, phenyl, benzyl, acyl of l to 4 carbon atoms, phenacyl;
- R¹⁶: is H, alkyl of l to 6 carbon atoms, cycloalkyl of 3 to 6 carbon atoms, (CH₂)ₚC₆H₅, OR¹⁷, or NR¹⁸R¹⁹;
- R¹⁷: is H, alkyl of 1 to 6 carbon atoms, cycloalkyl of 3 to 6 carbon atoms, phenyl or benzyl;
- R¹⁸ and R¹⁹: independently are H, alkyl of l to 4 carbon atoms, phenyl, benzyl, α-methylbenzyl, or taken together with the nitrogen form a ring of the formula 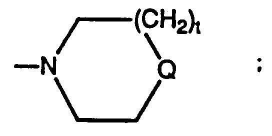
- Q: is NR²⁰, O or CH₂;
- R²⁰: is H, alkyl of l-4 carbon atoms, or phenyl;
- R²¹: is alkyl of 1 to 6 carbon atoms, -NR²²R²³, or 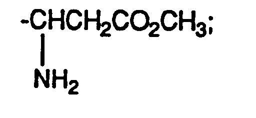
- R²² and R²³: independently are H, alkyl of 1 to 6 carbon atoms, benzyl, or are taken together as (CH₂)ᵤ where u is 3-6;
- R²⁴: is H, CH₃ or -C₆H₅;
- R²⁵: is NR²⁷R²⁸, OR²⁸, NHCONH₂, NHCSNH₂, 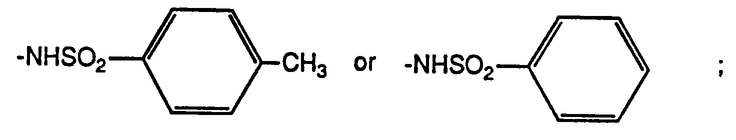
- R²⁶: is hydrogen, alkyl with from 1 to 6 carbon atoms, benzyl, or allyl;
- R²⁷ and R²⁸: are independently hydrogen, alkyl with from 1 to 5 carbon atoms, or phenyl;
- R²⁹ and R³⁰: are independently alkyl of 1-4 carbon atoms or taken together are -(CH₂)_{q}-;
- R³¹: is H, alkyl of 1 to 4 carbon atoms, -CH₂CH=CH₂ or -CH₂C₆H₄R³²;
- R³²: is H, NO₂, NH₂, OH or OCH₃;
- X: is a carbon-carbon single bond, -CO-, -CH₂-, -O-, -S-, -NH-, 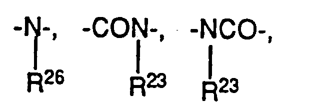 -OCH₂-, -CH₂O-, -SCH₂-, -CH₂S-, -NHC(R²⁷)(R²⁸)-, -NR²³SO₂-, -SO₂NR²³-, -C(R²⁷)(R²⁸)NH-, -CH=CH-, -CF=CF-, -CH=CF-, -CF=CH-, -CH₂CH₂-, -CF₂CF₂-, 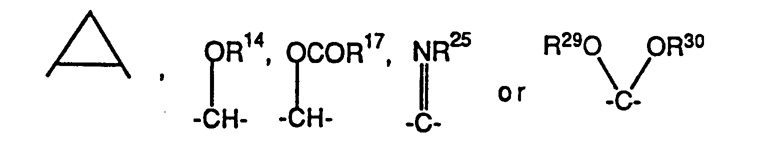
- Y: is O or S;
- Z: is O, NR¹¹, or S;
- m: is 1 to 5;
- n: is 1 to 10;
- p: is 0 to 3;
- q: is 2 to 3;
- r: is 0 to 2;
- s: is 0 to 5;
- t: is 0 or 1;
or a pharmaceutically acceptable salt thereof;
provided that:
(1) the R¹ group is not in the ortho position;
(2) when R¹ is 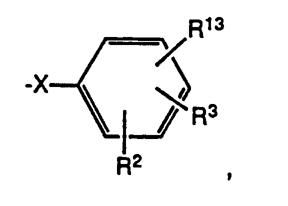
- X: is a single bond, and R¹³ is CO₂H, or 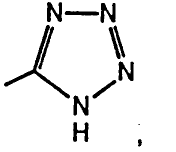 then R¹³ must be in the ortho or meta position; or when R¹ and X are as above and R¹³ is NHSO₂CF₃ or NHSO₂CH₃, R¹³ must be ortho;
(3) when R¹ is 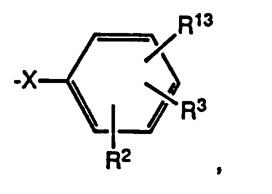
and X is other than a single bond, then R¹³ must be ortho except when X = NR²³CO and R¹³ is NHSO₂CF₃ or NHSO₂CH₃, then R¹³ must be ortho or meta;
(4) when R¹ is 4-CO₂H or a salt thereof, R⁶ cannot be S-alkyl;
(5) when R¹ is 4-CO₂H or a salt thereof, the substituent on the 4-position of the imidazole cannot be CH₂OH, CH₂OCOCH₃, or CH₂CO₂H;
(6) when R¹ is 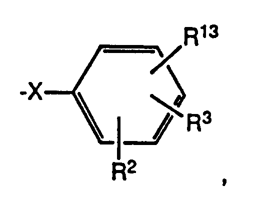
- X: is -OCH₂-, and R¹³ is 2-CO₂H, and R⁷ is H then R⁶ is not C₂H₅S;
(7) when R¹ is 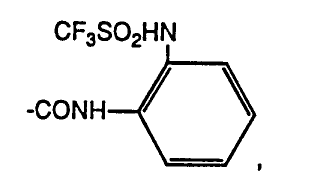
and R⁶ is n-hexyl, then R⁷ and R⁸ are not both hydrogen;
(8) when R¹ is 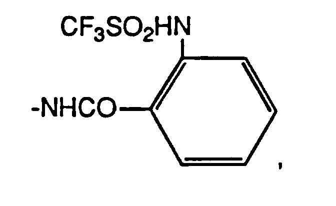
- R⁶: is not methoxybenzyl;
(9) the R⁶ group is not -CHFCH₂CH₂CH₃ or CH₂OH;
(10) when r=O, R¹ is 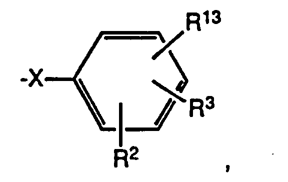
X is -NH-CO-, R¹³ is 2-NHSO₂CF₃, and R⁶ is n-propyl, then R⁷ and R⁸ are not -CO₂CH₃;
(11) when r=0, R¹ is 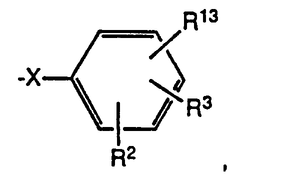
X is -NH-CO-, R¹³ is 2-COOH, and R⁶ is n-propyl, then R⁷ and R⁸ are not -CO₂CH₃;
(12) when r=1, R¹= 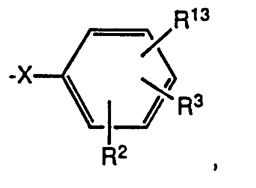
X is a single bond, R⁷ is Cl, and R⁸ is -CHO, then R¹³ is not 3-(tetrazol-5-yl);
(13) when r=1, R¹= 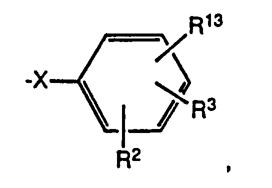
X is a single bond, R⁷ is Cl, and R⁸ is -CHO, then R¹³ is not 4-(tetrazol-5-yl) for preparing a medicament for treating chronic renal failure mediated by angiotensin II.

Preferred in the use of this invention are compounds having the formula (II): 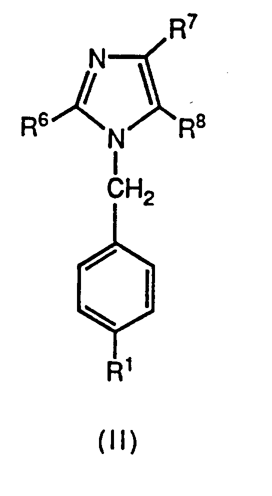 wherein
- R¹: is -CO₂H; -NHSO₂CF₃; 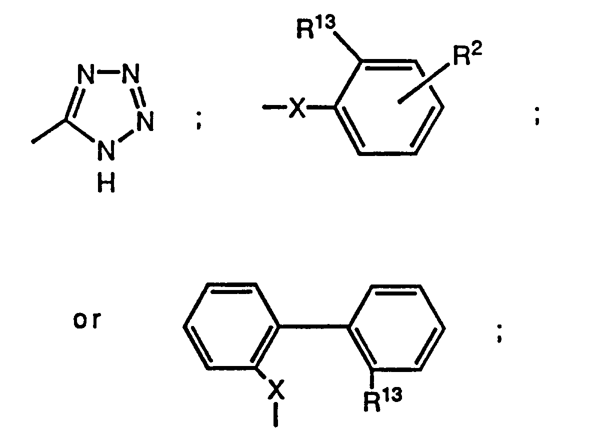
- R⁶: is alkyl of 3 to 10 carbon atoms, alkenyl of 3 to 10 carbon atoms, alkynyl of 3 to 10 carbon atoms, cycloalkyl of 3 to 8 carbon atoms, benzyl substituted on the phenyl ring with up to two groups selected from alkoxy of 1 to 4 carbon atoms, halogen, alkyl of 1 to 4 carbon atoms, and nitro;
- R⁸: is -(CH₂)ₛ-tetrazolyl, -(CH₂)ₙOR¹¹; 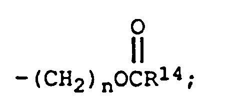 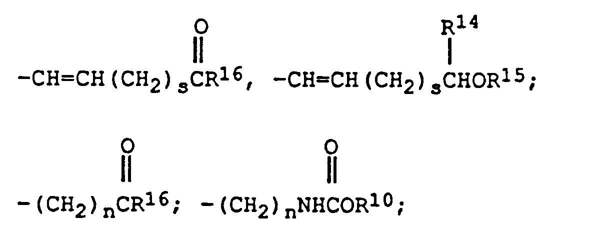 -(CH₂)ₙNHSO₂R¹⁰; -(CH₂)ₘF; or 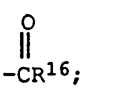 phenylalkenyl wherein the aliphatic portion is 2 to 4 carbon atoms; -(CH₂)ₘ-imidazol-l-yl; or -(CH₂)ₘ-1,2,3-triazolyl optionally substituted with one two groups selected from -CO₂CH₃ or alkyl or 1 to 4 carbon atoms;
- R¹³: is -CO₂H, -CO₂R⁹, NHSO₂CF₃; SO₃H; or 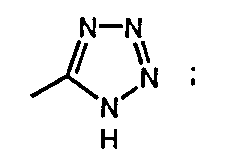
- R¹⁶: is H, alkyl or 1 to 5 carbon atoms, OR¹⁷, or NR¹⁸R¹⁹;
- X: is carbon-carbon single bond, -CO-, -CH₂CH₂-, 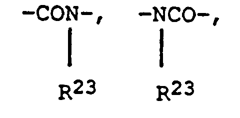 -OCH₂-, -CH₂O-, -O-, -SCH₂-, -CH₂S-, -NH-CH₂-, -CH₂NH- or -CH=CH-; and pharmaceutically acceptable salts of these compounds.

More preferred in the use of the invention are compounds of the preferred scope where:
- R²: is H, alkyl of 1 to 4 carbon atoms, halogen, or alkoxy or 1 to 4 carbon atoms;
- R⁶: is alkyl, alkenyl or alkynyl of 3 to 7 carbon atoms;
- R⁷: is heteroaryl selected from 2- and 3-thienyl, 2-and 3-furyl, 2-, 3-, and 4-pyridyl, p-biphenylyl; H, Cl, Br, I; CᵥF₂ᵥ₊₁, where v=1-3; 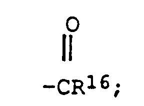 straight or branched chain alkyl of 1 to 6 carbon atoms; or phenyl;
- R⁸: is -(CH₂)ₘOR¹¹; 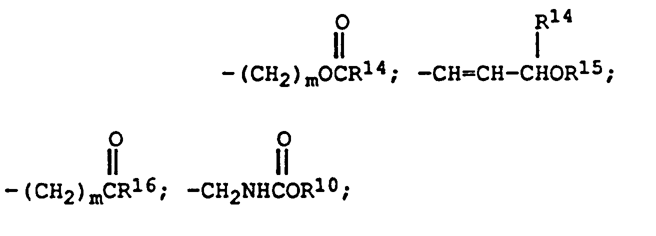 -(CH₂)ₘNHSO₂R¹⁰; 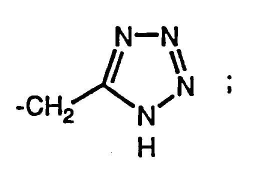 or -COR¹⁶;
- R¹⁰: is CF₃, alkyl or 1 to 6 carbon atoms or phenyl;
- R¹¹: is H, or alkyl or 1 to 4 carbon atoms;
- R¹³: is CO₂H; CO₂CH₂OCOC(CH₃)₃; NHSO₂CF₃; 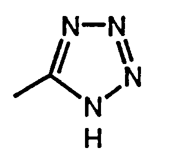
- R¹⁴: is H, or alkyl of 1 to 4 carbon atoms;
- R¹⁵: is H, alkyl or 1 to 4 carbon atoms, or acyl or 1 to 4 carbon atoms;
- R¹⁶: is H, alkyl or 1 to 5 carbon atoms; OR¹⁷; or 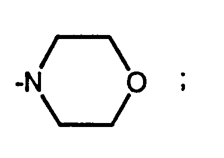
- m: is 1 to 5;
- X: = single bond, -O-; -CO-; -NHCO-; or -OCH₂-; and pharmaceutically acceptable salts.

More preferred in the use of the invention are compounds of Formula II, wherein R¹ is 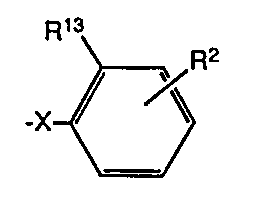 and X is a single bond; and pharmaceutically suitable salts thereof.

Most preferred in the use of the invention are compounds of formula II selected from the following, and pharmaceutically acceptable salts thereof:
- 2-Butyl-4-chloro-1-[(2'-(1H-tetrazol-5-yl)biphenyl-4-yl)methyl]-5-(hydroxymethyl)imidazole
- 2-Butyl-4-chloro-1-[(2'-carboxybiphenyl-4-yl)methyl]-5-(hydroxymethyl)imidazole
- 2-Butyl-4-chloro-1-[(2'-carboxybiphenyl-4-yl)methyl]-5-[(methoxycarbonyl)aminomethyl] imidazole
- 2-Butyl-4-chloro-1-[(2'-carboxybiphenyl-4-yl)methyl]-5-[(propoxycarbonyl)aminomethyl] imidazole
- 2-Butyl-4-chloro-1-[(2'-carboxybiphenyl-4-yl)methyl]imidazole-5-carboxaldehyde
- 2-Butyl-1-[(2'-carboxybiphenyl-4-yl)methyl]imidazole-5-carboxaldehyde
- 2-(1E-Butenyl)-4-chloro-1-[(2'-carboxybiphenyl-4-yl)methyl]-5-(hydroxymethyl)imidazole
- 2-(1E-Butenyl)-4-chloro-1-[(2'-carboxybiphenyl-4-yl)methyl]imidazole-5-carboxaldehyde
- 2-Propyl-4-chloro-1-[(2'-(1H-tetrazol-5-yl)biphenyl-4-yl)methyl]-5-(hydroxymethyl)imidazole
- 2-Propyl-4-chloro-1-[(2'-(1H-tetrazol-5-yl)biphenyl-4-yl)methyl]imidazole-5-carboxaldehyde
- 2-Butyl-4-chloro-1-[2'-(1H-tetrazol-5-yl)biphenyl-4-yl)methyl]imidazole-5-carboxaldehyde
- 2-(1E-Butenyl)-4-chloro-1-[(2'-(1H-tetrazol-5-yl)biphenyl-4-yl)methyl]-5-hydroxymethyl)imidazole
- 2-(1E-Butenyl)-4-chloro-1-[(2'-(1H-tetrazol-5-yl)biphenyl-4-yl)methyl]imidazole-5-carboxaldehyde
- 2-Butyl-4-chloro-1-[(2'-(1H-tetrazol-5-yl)-biphenyl-4-yl)methyl]imidazole-5-carboxylic acid
- 2-Propyl-4-chloro-1-[(2'-(1H-tetrazol-5-yl)biphenyl-4-yl)methyl]imidazole-5-carboxylic acid
- 2-Propyl-4-trifluoromethyl-1-[(2'-(1H-tetrazol-5-yl)biphenyl-4-yl)methyl]imidazole-5-carboxylic acid
- 2-Propyl-4-trifluoromethyl-1-[(2'-(1H-tetrazol-5-yl)biphenyl-4-yl)methyl]-5-(hydroxylmethyl)imidazole
- 2-Butyl-4-trifluoromethyl-1-[(2'-(1H-tetrazol-5-yl)biphenyl-4-yl)methyl]imidazole-5-carboyxlic acid
- 2-Propyl-4-trifluoromethyl-1-[(2'-(carboxybiphenyl-4-yl)methyl]imidazole-5-carboxaldehyde
- 2-Propyl-4-pentafluoroethyl-1-[(2'-(1H-tetrazol-5-yl)biphenyl-4-yl)methyl]-5-(hydroxymethyl)-imidazole
- 2-Propyl-1-[(2'-(1H-tetrazol-5-yl)biphenyl-4-yl)methyl]imidazole-4,5-dicarboxylic acid
- 2-Propyl-4-pentafluoroethyl-1-[(2'-(1H-tetrazol-5-yl)biphenyl-4-yl)methyl]imidazole-5-carboxylic acid
- 2-Propyl-4-pentafluoroethyl-[(2'-(1H-tetrazol-5-yl)biphenyl-4-yl)methyl]imidazole-5-carboxaldehyde
- 1-[(2'-(1H-Tetrazol-5-yl)biphenyl-4-yl)methyl]-4-phenyl-2-propylimidazole-5-carboxaldehyde
- 1-[(2'-Carboxybiphenyl-4-yl)methyl]-4-phenyl-2-propylimidazole-5-carboxaldehyde

Note that throughout the text when an alkyl substituent is mentioned, the normal alkyl structure is meant (i.e., butyl is n-butyl) unless otherwise specified.

Pharmaceutically suitable salts include both the metallic (inorganic) salts and organic salts; a list of which is given in Remington's Pharmaceutical Sciences, 17th Edition, pg. 1418 (1985). It is well known to one skilled in the art that an appropriate salt form is chosen based on physical and chemical stability, flowability, hydroscopicity and solubility. Preferred salts of this invention for the reasons cited above include potassium, sodium, calcium and ammonium salts.

The medicament can optionally contain one or more other therapeutic agents. It should be noted in the foregoing structural formula, when a radical can be a substituent in more than one previously defined radical, that first radical can be selected independently in each previously defined radical. For example, R¹, R² and R³ can each be CONHOR¹². R¹² need not be the same substituent in each of R¹, R² and R³ but can be selected independently for each of them.

### Detailed Description of the Invention

The compounds of Formula (I) useful in this invention are described in and prepared by methods set forth in EP-A-0 324 377, published 7/19/89, (page 17, line 5 through page 212, line 32), EP-A-0 253 310, published 1/20/88 (page 15, line 26, through page 276) and WO 91/00277, published 1/10/91 (page 16, line 21 through page 153, line 15).

It is believed that the compounds described herein are efficacious in the treatment of most chronic renal failure cases mediated by AII. While it is not clear, there is a possibility that the efficacy of these compounds may be limited in the treatment of renal artery stenosis due to a reversible loss of filtration in the stenotic kidney.

The following illustrate the use of nonpeptide AII receptor antagonists to treat chronic renal failure mediated by angiotensin-II:

### Partially Nephrectomized Rats

Rats are subjected to five-sixths renal ablation by surgically removing the right kidney and infarction of about two-thirds of the left kidney by ligation of two or three branches of the left renal artery as described by Anderson et al. in J. Clin. Invest., Vol. 76, pages 612-619 (August 1985). Rats are fed standard rat chow containing about 24% protein by weight and are separated into two groups. The rats in Group I are not treated. The rats in Group II are treated over a period of four weeks using one of the compounds described above. Renal hemodynamics and glomerular injury are monitored in both groups of rats after four weeks.

It is expected that the rats in Group I (no treatment) would have high blood pressure, protein urea and glomerular structural lesions whereas the rats in Group II (treatment with the test compound) would have normal blood pressure, less protein urea and fewer glomerular lesions.

### Streptozotocin-Induced Diabetic Rats

This procedure is described by Zatz et al. in Proc. Natl. Acad. Sci. USA 82: 5963-5967 (September 1985). Rats are studied two to ten weeks after being injected once with streptozotocin (60 mg/kg i.v.). In addition, ultralente insulin is given to maintain the blood glucose level between 200-400 mg/dl. Rats are maintained on diet containing about 50% protein by weight. The rats are then separated into two groups. The rats in Group I are not treated. The Group II rats are treated for about four to five weeks after streptozotocin injection using one of the compounds described above. Renal hemodynamics and glomerular injury are monitored in both groups of rats.

It is expected that the rats in Group I (no treatment) would have normal blood pressure, protein urea, and glomerular structural lesions whereas the rats in Group II (treatment with the test compound) would have slightly lower blood pressure, less protein urea and fewer glomerular lesions.

### DOSAGE FORMS

The compounds of this invention can be administered for the treatment of AII-mediated chronic renal failure according to the invention by any means that effects contact of the active ingredient compound with the site of action. For example, administration can be parenteral, i.e., subcutaneous, intravenous, intramuscular, or intraperitoneal. Alternatively, or concurrently, in some cases administration can be by the oral route.

The compounds can be administered by any conventional means available for use in conjunction with pharmaceuticals, either as individual therapeutic agents or in a combination of therapeutic agents. They can be administered alone, but are generally administered with a pharmaceutical carrier selected on the basis of the chosen route of administration and standard pharmaceutical practice.

For the purpose of this disclosure, a warm-blooded animal is a member of the animal kingdom possessed of a homeostatic mechanism and includes mammals and birds.

The dosage administered will be dependent on the age, health and weight of the recipient, the extent of disease, kind of concurrent treatment, if any, frequency of treatment and the nature of the effect desired. Usually, a daily dosage of active ingredient compound will be from 1-500 milligrams per day. Ordinarily, from 10 to 100 milligrams per day in one or more applications is effective to obtain desired results.

The active ingredient can be administered orally in solid dosage forms, such as capsules, tablets, and powders, or in liquid dosage forms, such as elixirs syrups, and suspensions. It can also be administered parenterally, in sterile liquid dosage forms.

Gelatin capsules contain the active ingredient and powdered carriers, such as lactose, starch, cellulose derivatives, magnesium stearate and stearic acid.

Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl- or propylparaben, and chlorobutanol.

Suitable pharmaceutical carriers are described in Remington's Pharmaceutical Sciences, A. Osol, a standard reference text in this field.

Useful pharmaceutical dosage-forms for administration of the compounds of this invention can be illustrated as follows:

### Capsules:

A large number of unit capsules are prepared by filling standard two-piece hard gelatin capsules each with 100 milligrams of powdered active ingredient, 150 milligrams of lactose, 50 milligrams of cellulose, and 6 milligrams magnesium stearate.

### Soft Gelatin Capsules:

A mixture of active ingredient in a digestible oil such as soybean oil, cottonseed oil or olive oil is prepared and injected by means of a positive displacement pump into gelatin to form soft gelatin capsules containing 100 milligrams of the active ingredient. The capsules are washed and dried.

### Tablets:

A large number of tablets are prepared by conventional procedures so that the dosage unit is 100 milligrams of active ingredient, 0.2 milligrams of colloidal silicon dioxide, 5 milligrams of magnesium stearate, 275 milligrams of microcrystalline cellulose, 11 milligrams of starch and 98.8 milligrams of lactose. Appropriate coatings may be applied to increase palatability or delay absorption.

### Injectable:

A parenteral composition suitable for administration by injection is prepared by stirring 1.5% by weight of active ingredient in 10% by volume propylene glycol. The solution is made to volume with water for injection and sterilized.

### Suspension:

An aqueous suspension is prepared for oral administration so that each 5 milliliters contain 100 milligrams of finely divided active ingredient, 100 milligrams of sodium carboxymethyl cellulose, 5 milligrams of sodium benzoate, 1.0 grams of sorbitol solution, U.S.P., and 0.025 milliliters of vanillin.

The same dosage forms can generally be used when the compounds of this invention are administered stepwise in conjunction with another therapeutic agent. When the drugs are administered in physical combination, the dosage form and administration route should be selected for compatibility with both drugs.

## Claims

1. Use of a compound having the formula (I): 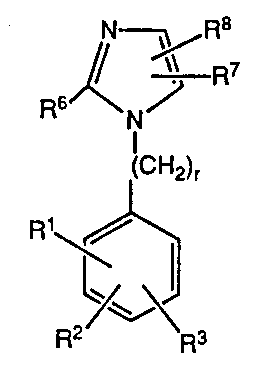 wherein
R¹ is 4-CO₂H; 4-CO₂R⁹; 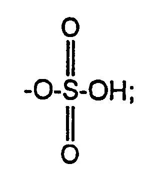 -SO₃H; -C(CF₃)₂OH; 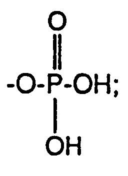 -PO₃H₂; 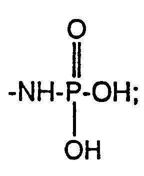 4-NHSO₂CH₃; -4-NHSO₂CF₃; -CONHOR¹²; -SO₂NH₂; 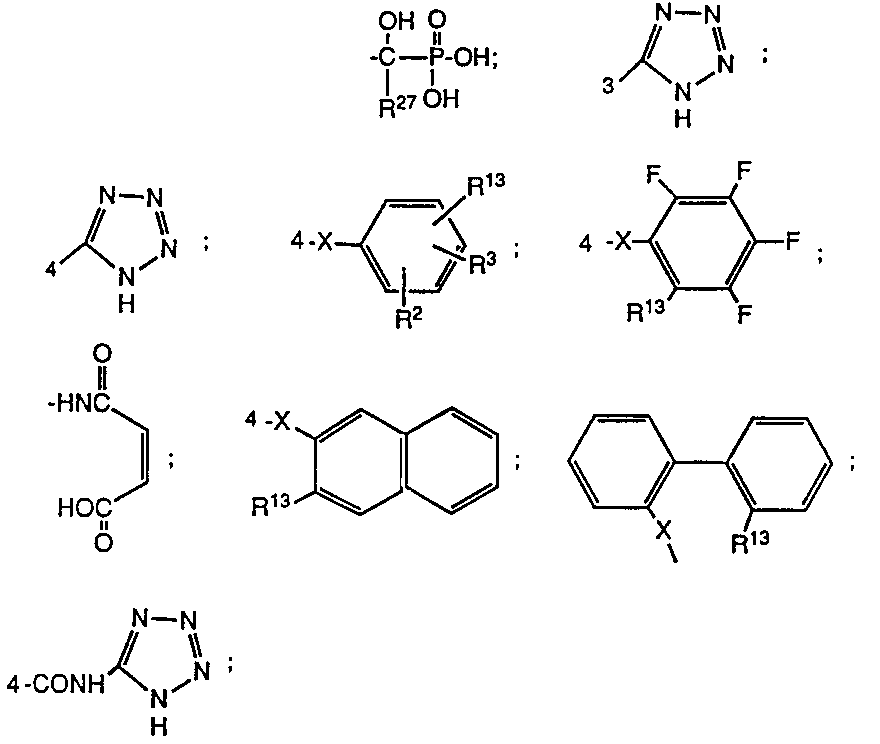
4-CONHNHSO₂CF₃; 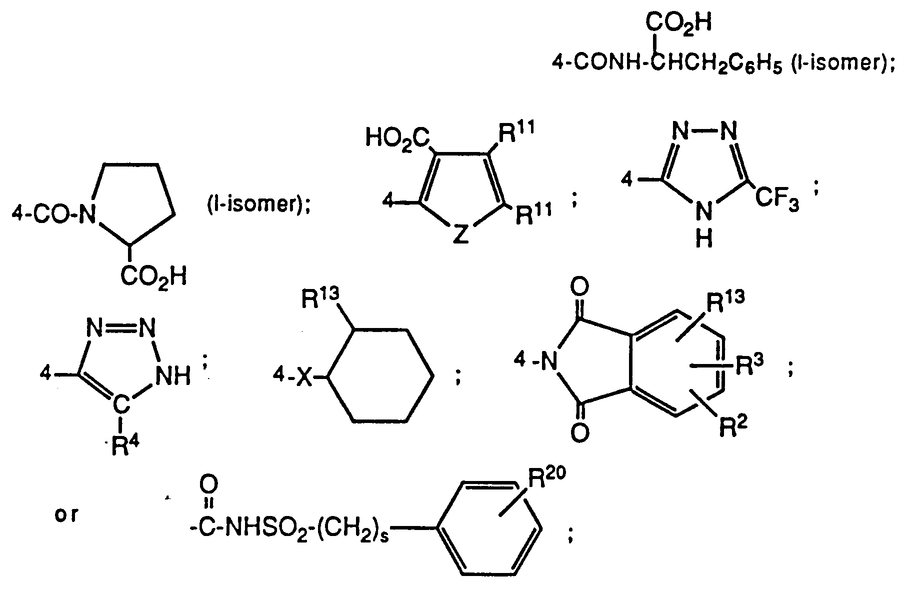
R² is H; Cl; Br; I; F; NO₂; CN; alkyl of 1 to 4 carbon atoms; acyloxy of 1 to 4 carbon atoms; alkoxy of 1 to 4 carbon atoms; CO₂H; CO₂R⁹; NHSO₂CH₃; NHSO₂CF₃; CONHOR¹²; SO₂NH₂; 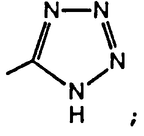 aryl; or furyl;
R³ is H; Cl, Br, I or F; alkyl of 1 to 4 carbon atoms or alkoxy of 1 to 4 carbon atoms;
R⁴ is CN, NO₂ or CO₂R¹¹;
R⁵ is H, alkyl of 1 to 6 carbon atoms, cycloalkyl of 3 to 6 carbon atoms, alkenyl or alkynyl of 2 to 4 carbon atoms;
R⁶ is alkyl of 2 to 10 carbon atoms, alkenyl or alkynyl of 3 to 10 carbon atoms or the same groups substituted with F or CO₂R¹⁴; cycloalkyl of 3 to 8 carbon atoms, cycloalkylalkyl of 4 to 10 carbon atoms; cycloalkylalkenyl or cycloalkylalkynyl of 5 to 10 carbon atoms; (CH₂)ₛZ(CH₂)ₘR⁵ optionally substituted with F or CO₂R¹⁴; benzyl or benzyl substituted on the phenyl ring with 1 or 2 halogens, alkoxy of 1 to 4 carbon atoms, alkyl of 1 to 4 carbon atoms or nitro;
R⁷ is H, F, Cl, Br, I, NO₂, CᵥF₂ᵥ₊₁, where v = 1-6; C₆F₅; CN; 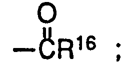 straight or branched alkyl of 1 to 6 carbon atoms; phenyl or phenylalkyl, where alkyl is 1 to 3 carbon atoms; or substituted phenyl or substituted phenylalkyl, where alkyl is 1 to 3 carbon atoms, substituted with one or two substituents selected from alkyl of 1 to 4 carbon atoms, F, Cl, Br, OH, OCH₃, CF₃, and COOR, where R is H, alkyl of 1 to 4 carbon atoms, or phenyl; vinyl; alkynyl of 2-10 carbon atoms; phenylalkynyl where the alkynyl portion is 2-6 carbon atoms; heteroaryl selected from 2- and 3-thienyl, 2- and 3-furyl, 2-, 3-, and 4-pyridyl, 2-pyrazinyl, 2-, 4-, and 5-pyrimidinyl, 3- and 4-pyridazinyl, 2-, 4- and 5-thiazolyl, 2-, 4-, and 5-selenazolyl, 2-, 4-, and 5-oxazolyl; 2- or 3-pyrrolyl, 3-, 4- or 5-pyrazolyl, and 2-, 4- or 5-imidazolyl; o-, m- or p-biphenylyl; o-, m- or p-phenoxyphenyl; substituted phenylalkynyl, heteroaryl, biphenyl or phenoxyphenyl as defined above substituted on ring carbon with 1 or 2 substituents selected from halogen, alkoxy of 1-5 carbon atoms, alkyl of 1-5 carbon atoms, -NO₂, -CN, -CF₃, -COR¹⁶, -CH₂OR¹⁷, -NHCOR¹⁷, CONR¹⁸R¹⁹, S(O)ᵣR¹⁷, and SO₂NR¹⁸R¹⁹; pyrrolyl, pyrazolyl or imidazolyl as defined above substituted on ring nitrogen with alkyl of 1-5 carbon atoms or benzyl; or substituted alkyl, alkenyl, or alkynyl of 1 to 10 carbon atoms substituted with a substituted or unsubstituted heteroaryl, biphenylyl or phenoxyphenyl group as defined above; any of the foregoing polycyclic aryl groups substituted with 1 or 2 substituents selected from halogen, alkoxy of 1-5 carbon atoms, alkyl of 1-5 carbon atoms, -NO₂, -CN, -CF₃, -COR¹⁶, -CH₂OR¹⁷, -NHCOR¹⁷, CONR¹⁸R¹⁹, S(O)ᵣR¹⁷, and SO₂NR¹⁸R¹⁹; the anhydride of 4,5-dicarboxyl-1- or 2-naphthyl; or substituted alkyl of 1 to 10 carbon atoms, alkenyl or alkynyl of 2 to 10 carbon atoms substituted with a substituted or unsubstituted polycyclic aryl group as defined above;
R⁸ is H, CN, alkyl of 1 to 10 carbon atoms, alkenyl of 3 to 10 carbon atoms, or the same groups substituted with F; phenylalkenyl wherein the aliphatic portion is 2 to 6 carbon atoms; -(CH₂)ₘ-imidazol-1-yl; -(CH₂)ₘ-1,2,3-triazolyl optionally substituted with one or two groups selected from CO₂CH₃ or alkyl of l to 4 carbon atoms; -(CH₂)ₛ-tetrazolyl; 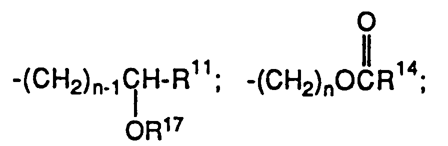 -(CH₂)ₙSR¹⁵; 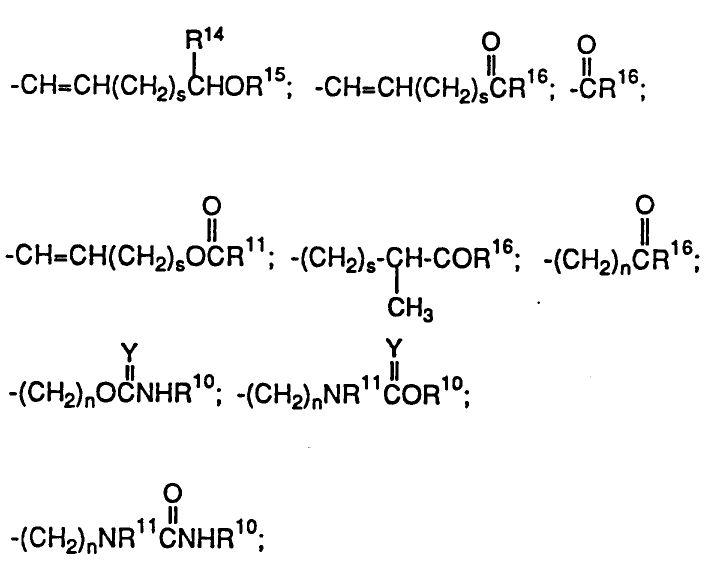 -(CH₂)ₙNR¹¹SO₂R¹⁰; 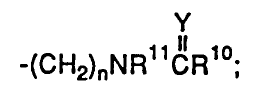 -(CH₂)ₘF; -(CH₂)ₘONO₂; -CH₂N_{3;} -(CH₂)ₘNO₂; -CH=N-NR¹¹R¹⁷; 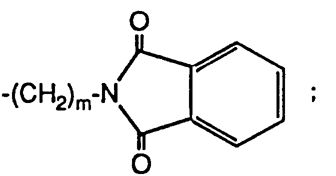 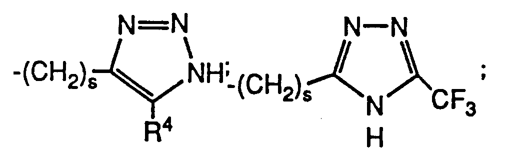 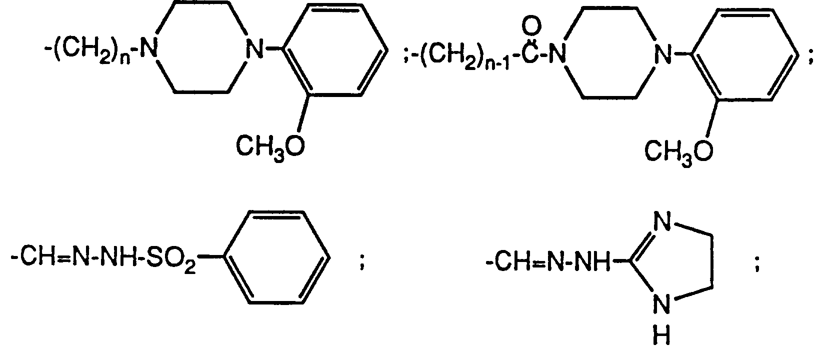
R⁹ is 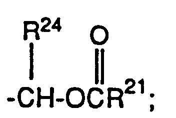
R¹⁰ is alkyl of 1 to 6 carbon atoms or perfluoroalkyl of 1 to 6 carbon atoms, 1-adamantyl, 1-naphthyl, 1-(1-naphthyl)ethyl, or (CH₂)ₚC₆H₅;
R¹¹ is H, alkyl of 1 to 6 carbon atoms, cycloalkyl of 3 to 6 carbon atoms, phenyl or benzyl;
R¹² is H, methyl or benzyl;
R¹³ is -CO₂H; -CO₂R⁹; -CH₂CO₂H, -CH₂CO₂R⁹; 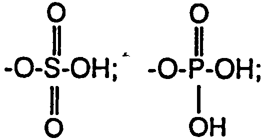 -SO₃H; 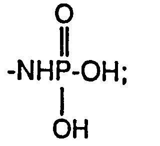 -PO₃H₂; -C(CF₃)₂OH; -NHSO₂CH₃; -NHSO₂CF₃; -NHCOCF₃; -CONHOR¹²; -SO₂NH₂; 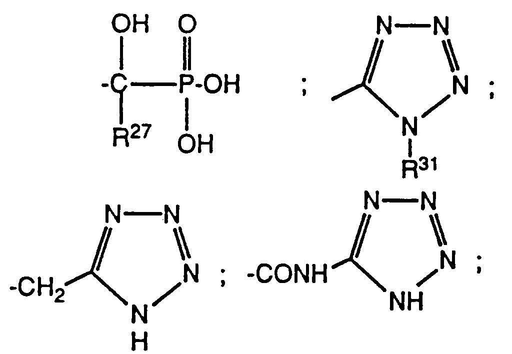 -CONHNHSO₂CF₃ ; 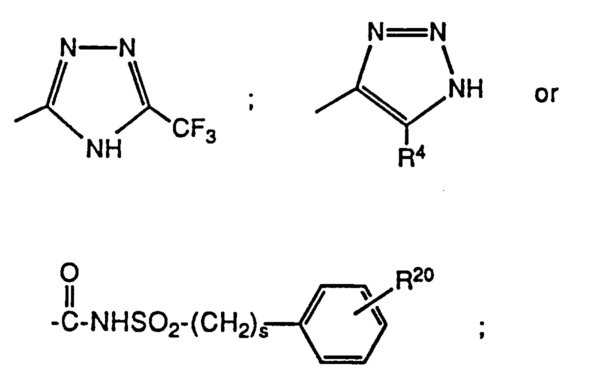
R¹⁴ is H, alkyl or perfluoroalkyl of 1 to 8 carbon atoms, cycloalkyl of 3 to 6 carbon atoms, phenyl or benzyl;
R¹⁵ is H, alkyl of 1 to 6 carbon atoms, cycloalkyl of 3 to 6 carbon atoms, phenyl, benzyl, acyl of l to 4 carbon atoms, phenacyl;
R¹⁶ is H, alkyl of l to 6 carbon atoms, cycloalkyl of 3 to 6 carbon atoms, (CH₂)ₚC₆H₅, OR¹⁷, or NR¹⁸R¹⁹;
R¹⁷ is H, alkyl of 1 to 6 carbon atoms, cycloalkyl of 3 to 6 carbon atoms, phenyl or benzyl;
R¹⁸ and R¹⁹ independently are H, alkyl of l to 4 carbon atoms, phenyl, benzyl, α-methylbenzyl, or taken together with the nitrogen form a ring of the formula 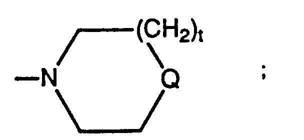
Q is NR²⁰, O or CH₂;
R²⁰ is H, alkyl of l-4 carbon atoms, or phenyl;
R²¹ is alkyl of 1 to 6 carbon atoms, -NR²²R²³, or 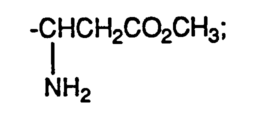
R²² and R²³ independently are H, alkyl of 1 to 6 carbon atoms, benzyl, or are taken together as (CH₂)ᵤ where u is 3-6;
R²⁴ is H, CH₃ or -C₆H₅;
R²⁵ is NR²⁷R²⁸, OR²⁸, NHCONH₂, NHCSNH₂, 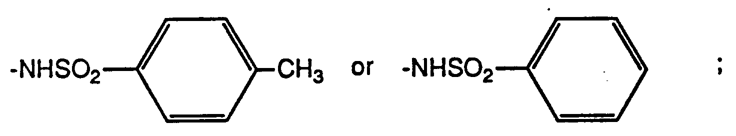
R²⁶ is hydrogen, alkyl with from 1 to 6 carbon atoms, benzyl, or allyl;
R²⁷ and R²⁸ are independently hydrogen, alkyl with from 1 to 5 carbon atoms, or phenyl;
R²⁹ and R³⁰ are independently alkyl of 1-4 carbon atoms or taken together are -(CH₂)_{q}-;
R³¹ is H, alkyl of 1 to 4 carbon atoms, -CH₂CH=CH₂ or -CH₂C₆H₄R³²;
R³² is H, NO₂, NH₂, OH or OCH₃;
X is a carbon-carbon single bond, -CO-, -CH₂-, -O-, -S-, -NH-, 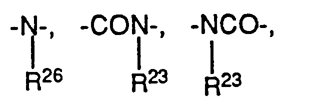 -OCH₂-, -CH₂O-, -SCH₂-, -CH₂S-, -NHC(R²⁷)(R²⁸)-, -NR²³SO₂-, -SO₂NR²³-, -C(R²⁷)(R²⁸)NH-, -CH=CH-, -CF=CF-, -CH=CF-, -CF=CH-, -CH₂CH₂-, -CF₂CF₂-, 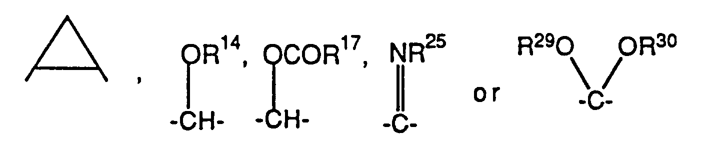
Y is O or S;
Z is O, NR¹¹, or S;
m is 1 to 5;
n is 1 to 10;
p is 0 to 3;
q is 2 to 3;
r is 0 to 2;
s is 0 to 5;
t is 0 or 1;
and pharmaceutically acceptable salt of these compounds;
provided that:
(1) the R¹ group is not in the ortho position;
(2) when R¹ is 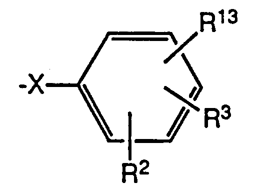 X is a single bond, and R¹³ is CO₂H, or 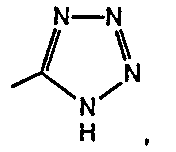 then R¹³ must be in the ortho or meta position; or when R¹ and X are as above and R¹³ is NHSO₂CF₃ or NHSO₂CH₃, R¹³ must be ortho;
(3) when R¹ is 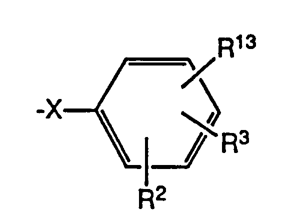 and X is other than a single bond, then R¹³ must be ortho except when X = NR²³CO and R¹³ is NHSO₂CF₃ or NHSO₂CH₃, then R¹³ must be ortho or meta;
(4) when R¹ is 4-CO₂H or a salt thereof, R⁶ cannot be S-alkyl;
(5) when R¹ is 4-CO₂H or a salt thereof, the substituent on the 4-position of the imidazole cannot be CH₂OH, CH₂OCOCH₃, or CH₂CO₂H;
(6) when R¹ is 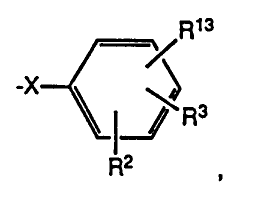
X is -OCH₂-, and R¹³ is 2-CO₂H, and R⁷ is H then R⁶ is not C₂H₅S;
(7) when R¹ is 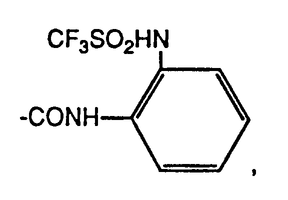
and R⁶ is n-hexyl, then R⁷ and R⁸ are not both hydrogen;
(8) when R¹ is 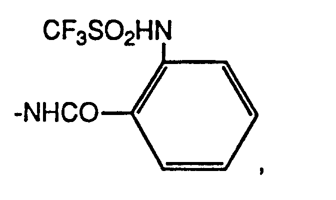
R⁶ is not methoxybenzyl;
(9) the R⁶ group is not -CHFCH₂CH₂CH₃ or CH₂OH;
(10) when r=O, R¹ is 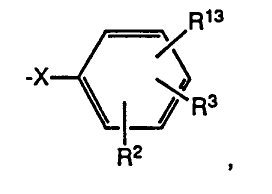
X is -NH-CO-, R¹³ is 2-NHSO₂CF₃, and R⁶ is n-propyl, then R⁷ and R⁸ are not -CO₂CH₃;
(11) when r=0, R¹ is 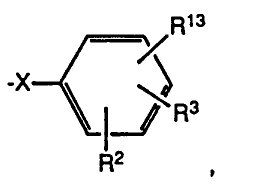
X is -NH-CO-, R¹³ is 2-COOH, and R⁶ is n-propyl, then R⁷ and R⁸ are not -CO₂CH₃;
(12) when r=1, R¹= 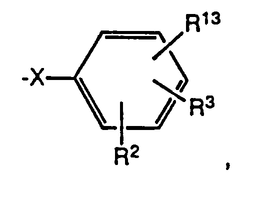
X is a single bond, R⁷ is Cl, and R⁸ is -CHO, then R¹³ is not 3-(tetrazol-5-yl);
(13) when r=1, R¹= 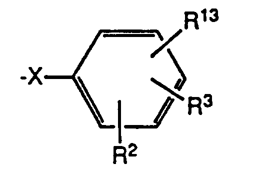
X is a single bond, R⁷ is Cl, and R⁸ is -CHO, then R¹³ is not 4-(tetrazol-5-yl)
for preparing a medicament for treating chronic renal failure mediated by angiotensin-II.

2. The use of claim 1 wherein the compound of formula I is a compound of formula II: 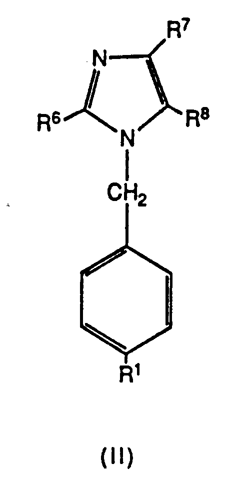 wherein R¹ is -CO₂H; -NHSO₂CF₃; 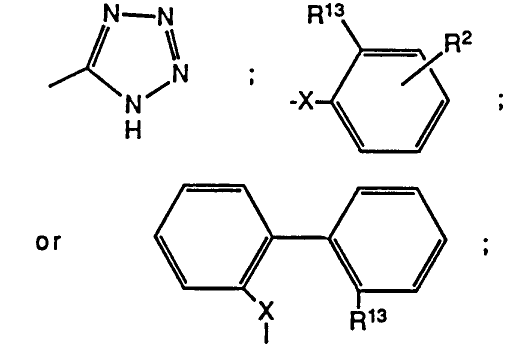
R⁶ is alkyl of 3 to 10 carbon atoms, alkenyl of 3 to 10 carbon atoms, alkynyl of 3 to 10 carbon atoms, cycloalkyl of 3 to 8 carbon atoms, benzyl substituted on the phenyl ring with up to two groups selected from alkoxy of 1 to 4 carbon atoms, halogen, alkyl of 1 to 4 carbon atoms, and nitro;
R⁸ is -(CH₂)ₛ-tetrazolyl, -(CH₂)ₙOR¹¹; 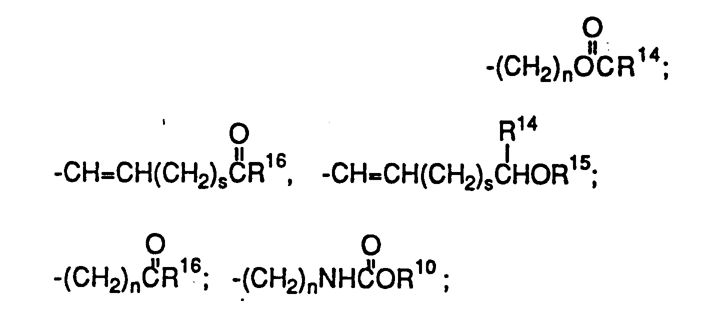 -(CH₂)ₙNHSO₂R¹⁰; -(CH₂)ₘF; or 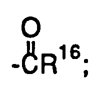
phenylalkenyl wherein the aliphatic portion is 2 to 4 carbon atoms; -(CH₂)ₘ-imidazol-l-yl; or -(CH₂)ₘ-1,2,3-triazolyl optionally substituted with one or two groups selected from -CO₂CH₃ or alkyl of 1 to 4 carbon atoms;
R¹³ is -CO₂H, -CO₂R⁹, NHSO₂CF₃; SO₃H; or 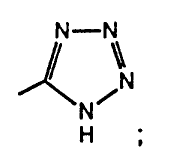
R¹⁶ is H, alkyl of 1 to 5 carbon atoms, OR¹⁷, or NR¹⁸R¹⁹;
X is carbon-carbon single bond, -CO-, -CH₂CH₂-, 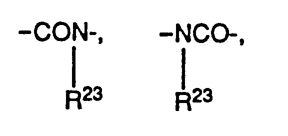
-OCH₂-, -CH₂O-, -O-, -SCH₂-, -CH₂S-, -NH-CH₂-, -CH₂NH-or -CH=CH-; or a pharmaceutically acceptable salt thereof.

3. The use of claim 2 wherein the compound of formula II is a compound wherein:
R² is H, alkyl of 1 to 4 carbon atoms, halogen, or alkoxy of 1 to 4 carbon atoms;
R⁶ is alkyl, alkenyl or alkynyl of 3 to 7 carbon atoms;
R⁷ is heteroaryl selected from 2- and 3-thienyl, 2- and 3-furyl, 2-, 3-, and 4-pyridyl, p-biphenylyl; H, Cl, Br, I; CᵥF₂ᵥ₊₁, where v=1-3; 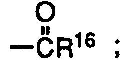 straight or branched chain alkyl of 1 to 6 carbon atoms; or phenyl;
R⁸ is -(CH₂)ₘOR¹¹; 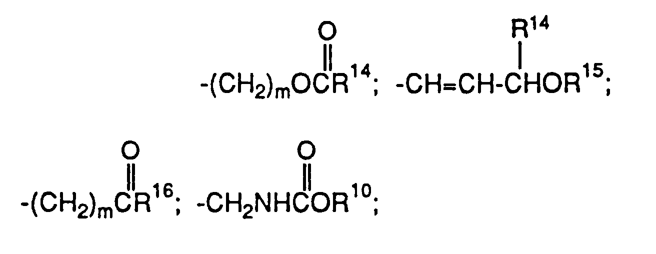 -(CH₂)ₘNHSO₂R¹⁰; 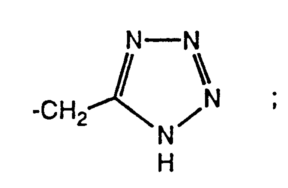 or -COR¹⁶;
R¹⁰ is CF₃, alkyl of l to 6 carbon atoms or phenyl;
R¹¹ is H, or alkyl of 1 to 4 carbon atoms;
R¹³ is CO₂H; CO₂CH₂OCOC(CH₃)₃; NHSO₂CF₃; 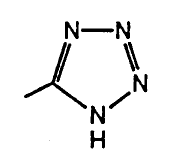
R¹⁴ is H, or alkyl of l to 4 carbon atoms;
R¹⁵ is H, alkyl of l to 4 carbon atoms, or acyl of l to 4 carbon atoms;
R¹⁶ is H, alkyl of 1 to 5 carbon atoms; OR¹⁷; or 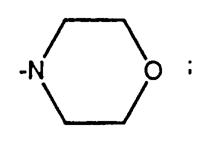
m is l to 5;
X = single bond, -O-; -CO-; -NHCO-; or -OCH₂-; or a pharmaceutically acceptable salt thereof.

4. The use of claim 3 wherein the compound of formula II is a compound wherein R¹ is 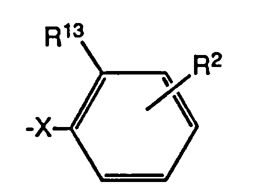 and X is a single bond; or a pharmaceutically suitable salt thereof.

5. The use of claim 4 wherein the compound of formula II is 2-Butyl-4-chloro-1-[(2'-(1H-tetrazol-5-yl)biphenyl-4-yl)methyl]-5-(hydroxymethyl)imidazole or a pharmaceutically acceptable salt thereof.

6. The use of claim 4 wherein the compound of formula II is 2-Butyl-4-chloro-1-[(2'-carboxybiphenyl-4-yl)methyl]-5-(hydroxymethyl)imidazole or a pharmaceutically acceptable salt thereof.

7. The use of claim 4 wherein the compound of formula II is 2-Butyl-4-chloro-1-[(2'-carboxybiphenyl-4-yl)methyl]-5-[(methoxycarbonyl)aminomethyl]imidazole or a pharmaceutically acceptable salt thereof.

8. The use of claim 4 wherein the compound of formula II is 2-Butyl-4-chloro-1-[(2'-carboxybiphenyl-4-yl)methyl]-5-[(propoxycarbonyl)aminomethyl]imidazole or a pharmaceutically acceptable salt thereof.

9. The use of claim 4 wherein the compound of formula II is 2-Butyl-4-chloro-1-[(2'-carboxybiphenyl-4-yl)methyl]imidazole-5-carboxaldehyde or a pharmaceutically acceptable salt thereof.

10. The use of claim 4 wherein the compound of formula II is 2-Butyl-1-[(2'-carboxybiphenyl-4-yl)methyl]-imidazole-5-carboxaldehyde or a pharmaceutically acceptable salt thereof.

11. The use of claim 4 wherein the compound of formula II is 2-(1E-Butenyl)-4-chloro-1-[(2'-carboxybiphenyl-4-yl)methyl]-5-(hydroxymethyl)imidazole or a pharmaceutically acceptable salt thereof.

12. The use of claim 4 wherein the compound of formula II is 2-(1E-Butenyl)-4-chloro-1-[(2'-carboxybiphenyl-4-yl)methyl]imidazole-5-carboxaldehyde or a pharmaceutically acceptable salt thereof.

13. The use of claim 4 wherein the compound of formula II is 2-Propyl-4-chloro-1-[(2'-(1H-tetrazol-5-yl)biphenyl-4-yl)methyl]-5-(hydroxymethyl)imidazole or a pharmaceutically acceptable salt thereof.

14. The use of claim 4 wherein the compound of formula II is 2-Propyl-4-chloro-1-[(2'-(1H-tetrazol-5-yl)biphenyl-4-yl)methyl]imidazole-5-carboxaldehyde or a pharmaceutically acceptable salt thereof.

15. The use of claim 4 wherein the compound of formula II is 2-Butyl-4-chloro-1-[2'-(1H-tetrazol-5-yl)biphenyl-4-yl)methyl]imidazole-5-carboxaldehyde or a pharmaceutically acceptable salt thereof.

16. The use of claim 4 wherein the compound of formula II is 2-(1E-Butenyl)-4-chloro-1-[(2'-(1H-tetrazol-5-yl)biphenyl-4-yl)methyl]-5- hydroxymethyl)imidazole or a pharmaceutically acceptable salt thereof.

17. The use of claim 4 wherein the compound of formula II is 2-(1E-Butenyl)-4-chloro-1-[(2'-(1H-tetrazol-5-yl)biphenyl-4-yl)methyl]imidazole-5-carboxaldehyde or a pharmaceutically acceptable salt thereof.

18. The use of claim 4 wherein the compound of formula II is 2-Butyl-4-chloro-1-[(2'-(1H-tetrazol-5-yl)-biphenyl-4-yl)methyl]imidazole-5-carboxylic acid or a pharmaceutically acceptable salt thereof.

19. The use of claim 4 wherein the compound of formula II is 2-Propyl-4-chloro-1-[(2'-(1H-tetrazol-5-yl)-biphenyl-4-yl)methyl]imidazole-5-carboxylic acid or a pharmaceutically acceptable salt thereof.

20. The use of claim 4 wherein the compound of formula II is 2-Propyl-4-trifluoromethyl-1-[(2'-(1H-tetrazol-5-yl)biphenyl-4-yl)methyl]imidazole-5-carboxylic acid or a pharmaceutically acceptable salt thereof.

21. The use of claim 4 wherein the compound of formula II is 2-Propyl-4-trifluoromethyl-1-[(2'-(1H-tetrazol-5-yl)biphenyl-4-yl)methyl]-5-(hydroxylmethyl)imidazole or a pharmaceutically acceptable salt thereof.

22. The use of claim 4 wherein the compound of formula II is 2-Butyl-4-trifluoromethyl-1-[(2'-(1H-tetrazol-5-yl)biphenyl-4-yl)methyl]imidazole-5-carboxylic acid or a pharmaceutically acceptable salt thereof.

23. The use of claim 4 wherein the compound of formula II is 2-Propyl-4-trifluoromethyl-1-[(2'-(carboxybiphenyl-4-yl)methyl]imidazole-5-carboxaldehyde or a pharmaceutically acceptable salt thereof.

24. The use of claim 4 wherein the compound of formula II is 2-Propyl-4-pentafluoroethyl-1-[(2'-(1H-tetrazol-5-yl)biphenyl-4-yl)methyl]-5-(hydroxymethyl)imidazole or a pharmaceutically acceptable salt thereof.

25. The use of claim 4 wherein the compound of formula II is 2-Propyl-4-pentafluoroethyl-1-[(2'-(1H-tetrazol-5-yl)biphenyl-4-yl)methyl]imidazole-5-carboxylic acid or a pharmaceutically acceptable salt thereof.

26. The use of claim 4 wherein the compound of formula II is 2-Propyl-4-pentafluoroethyl-[(2'-(1H-tetrazol-5-yl)biphenyl-4-yl)methyl]imidazole-5-carboxaldehyde or a pharmaceutically acceptable salt thereof.

27. The use of claim 4 wherein the compound of formula II is 1-[(2'-(1H-Tetrazol-5-yl)biphenyl-4-yl)methyl]-4-phenyl-2-propylimidazole-5-carboxaldehyde or a pharmaceutically acceptable salt thereof.

28. The use of claim 4 wherein the compound of formula II is 1-[(2'-Carboxybiphenyl-4-yl)methyl]-4-phenyl-2-propylimidazole-5-carboxaldehyde or a pharmaceutically acceptable salt thereof.

## Patentansprüche

1. Verwendung einer Verbindung mit der Formel (I): 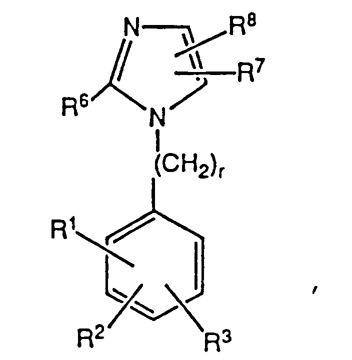 wobei
R¹ 4-CO₂H; 4-CO₂R⁹; 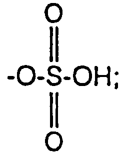 -SO₃H; -C(CF₃)₂OH; 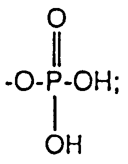 -PO₃H₂; 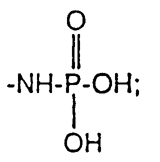 4-NHSO₂CH₃; -4-NHSO₂CF₃; -CONHOR¹²; -SO₂NH₂; 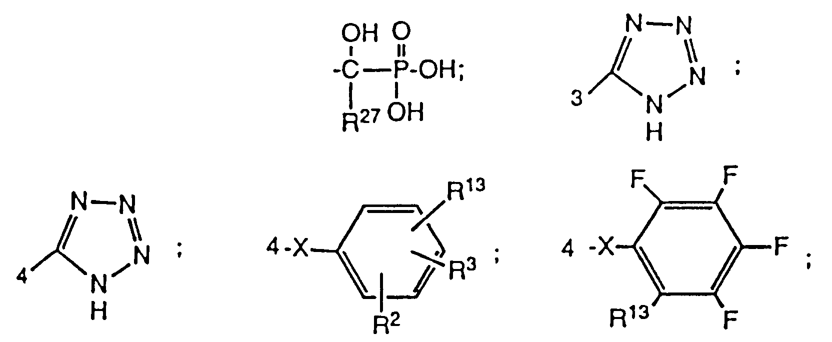 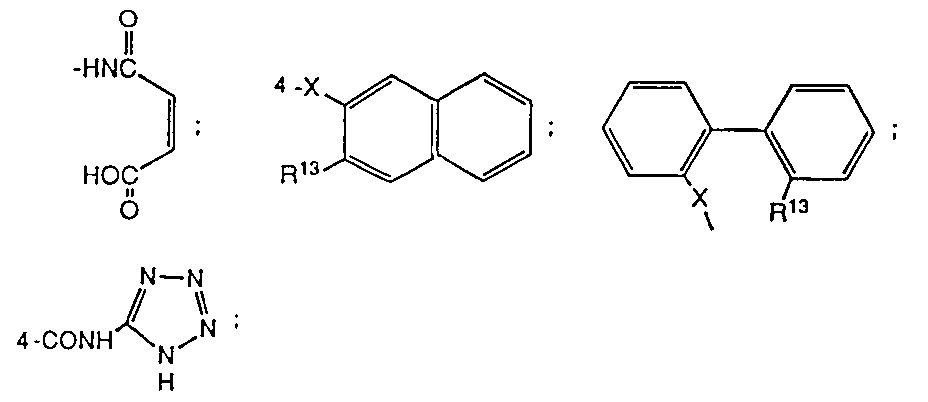 4-CONHNHSO₂CF₃; 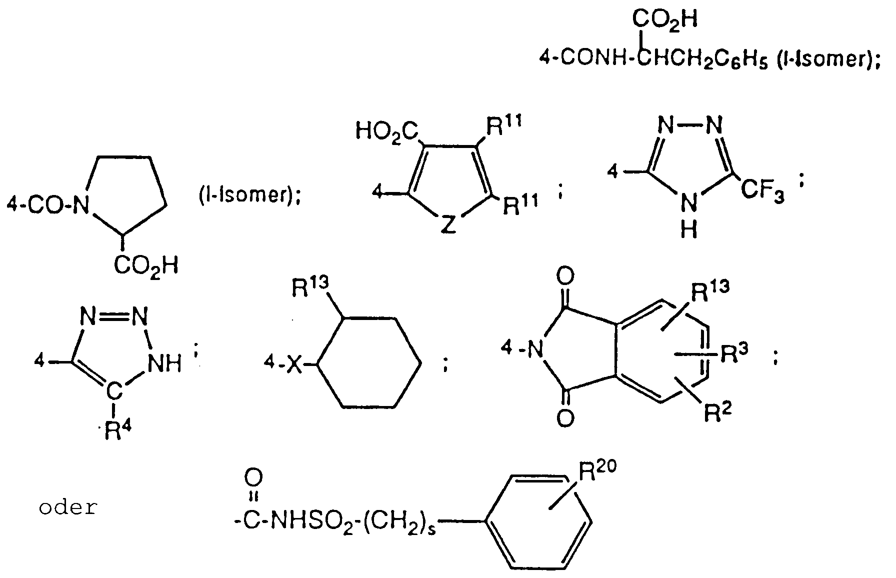 ist;
R² H; Cl; Br; I; F; NO₂; CN; ein Alkyl mit 1 bis 4 Kohlenstoffatomen; ein Acyloxy mit 1 bis 4 Kohlenstoffatomen; ein Alkoxy mit 1 bis 4 Kohlenstoffatomen; CO₂H; CO₂R⁹; NHSO₂CH₃; NHSO₂CF₃; CONHOR¹²; SO₂NH₂; 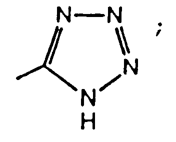 Aryl oder Furyl ist;
R³ H; Cl, Br, I oder F; ein Alkyl mit 1 bis 4 Kohlenstoffatomen oder ein Alkoxy mit 1 bis 4 Kohlenstoffatomen ist;
R⁴ CN, NO₂ oder CO₂R¹¹ ist;
R⁵ H, ein Alkyl mit 1 bis 6 Kohlenstoffatomen, ein Cycloalkyl mit 3 bis 6 Kohlenstoffatomen, ein Alkenyl oder Alkinyl mit 2 bis 4 Kohlenstoffatomen ist;
R⁶ ein Alkyl mit 2 bis 10 Kohlenstoffatomen, ein Alkenyl oder Alkinyl mit 3 bis 10 Kohlenstoffatomen oder dieselben, durch F oder CO₂R¹⁴ substituierten Gruppen; ein Cycloalkyl mit 3 bis 8 Kohlenstoffatomen; ein Cycloalkylalkyl mit 4 bis 10 Kohlenstoffatomen; ein Cycloalkylalkenyl oder Cycloalkylalkinyl mit 5 bis 10 Kohlenstoffatomen; (CH₂)ₛZ(CH₂)ₘR⁵, gegebenenfalls durch F oder CO₂R¹⁴ substituiert; Benzyl oder ein durch 1 oder 2 Halogene am Phenylring substituiertes Benzyl, ein Alkoxy mit 1 bis 4 Kohlenstoffatomen, ein Alkyl mit 1 bis 4 Kohlenstoffatomen oder Nitro ist;
R⁷ H, F, Cl, Br, I, NO₂, CᵥF₂ᵥ₊₁, wobei v = 1 - 6; C₆F₅;
CN; 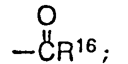 ein gerades oder verzweigtes Alkyl mit 1 bis 6 Kohlenstoffatomen; Phenyl oder ein Phenylalkyl, wobei das Alkyl 1 bis 3 Kohlenstoffatome aufweist; oder ein substituiertes Phenyl oder ein substituiertes Phenylalkyl, wobei das Alkyl 1 bis 3 Kohlenstoffatome aufweist, die durch ein oder zwei Substituenten, ausgewählt aus einem Alkyl mit 1 bis 4 Kohlenstoffatomen, F, Cl, Br, OH, OCH₃, CF₃ und COOR, wobei R H, ein Alkyl mit 1 bis 4 Kohlenstoffatomen ist, oder Phenyl substituiert sind; Vinyl; ein Alkinyl mit 2 - 10 Kohlenstoffatomen; ein Phenylalkinyl, wobei der Alkinylteil 2 - 6 Kohlenstoffatome aufweist; ein Heteroaryl, ausgewählt aus 2- und 3-Thienyl, 2- und 3-Furyl, 2-, 3- und 4-Pyridyl, 2-Pyrazinyl, 2-, 4- und 5-Pyrimidinyl, 3- und 4-Pyridazinyl, 2-, 4- und 5-Thiazolyl, 2-, 4- und 5-Selenazoloyl, 2-, 4- und 5-Oxazolyl; 2- oder 3-Pyrrolyl, 3-, 4- oder 5-Pyrazolyl und 2-, 4- oder 5-Imidazolyl; o-, m- oder p-Biphenylyl; o-, m- oder p-Phenoxyphenyl; ein wie oben definiertes, substituiertes Phenylalkinyl, Heteroaryl, Biphenyl oder Phenoxyphenyl, das am Ringkohlenstoff durch 1 oder 2 Substituenten, ausgewählt aus einem Halogen, einem Alkoxy mit 1 - 5 Kohlenstoffatomen, einem Alkyl mit 1-5 Kohlenstoffatomen, -NO₂, -CN, -CF₃, -COR¹⁶, -CH₂OR¹⁷, -NHCOR¹⁷, CONR¹⁸R¹⁹, S(O)ᵣR¹⁷ und SO₂NR¹⁸R¹⁹ substituiert ist; ein wie oben definiertes Pyrrolyl; Pyrazolyl oder Imidazolyl, das am Ringstickstoff durch ein Alkyl mit 1 - 5 Kohlenstoffatomen oder Benzyl substituiert ist; oder ein substituiertes Alkyl, Alkenyl oder Alkinyl mit 1 bis 10 Kohlenstoffatomen, das durch eine wie oben definierte, substituierte oder unsubstituierte Heteroaryl-, Biphenylyl- oder Phenoxyphenylgruppe substituiert ist; eine beliebige der vorhergehenden polycyclischen Arylgruppen, die durch 1 oder 2 Substituenten, ausgewählt aus einem Halogen, einem Alkoxy mit 1 bis 5 Kohlenstoffatomen, einem Alkyl mit 1 bis 5 Kohlenstoffatomen, -NO₂, -CN, -CF₃, -COR¹⁶, -CH₂OR¹⁷, -NHCOR¹⁷, CONR¹⁸R¹⁹, S(O)ᵣR¹⁷ und SO₂NR¹⁸R¹⁹, substituiert ist; das Anhydrid von 4,5-Dicarboxyl-1- oder - 2-naphthyl oder ein substituiertes Alkyl mit 1 bis 10 Kohlenstoffatomen, ein Alkenyl oder ein Alkinyl mit 2 bis 10 Kohlenstoffatomen ist, das durch eine wie oben definierte substituierte oder unsubstituierte polycyclische Gruppe substituiert ist;
R⁸ H, CN, ein Alkyl mit 1 bis 10 Kohlenstoffatomen, ein Alkenyl mit 3 bis 10 Kohlenstoffatomen oder dieselben, durch F substituierten Gruppen; ein Phenylalkenyl, wobei der aliphatische Teil 2 bis 6 Kohlenstoffatome aufweist; -(CH₂)ₘ-imidazol-1-yl; -(CH₂)ₘ-1,2,3-triazolyl, gegebenenfalls substituiert durch eine oder zwei Gruppen, ausgewählt aus CO₂CH₃ oder einem Alkyl mit 1 bis 4 Kohlenstoffatomen; -(CH₂)ₛ-tetrazolyl; 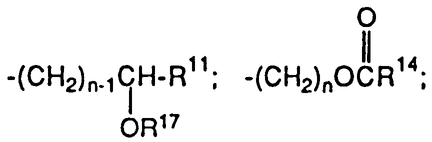 -(CH₂)ₙSR¹⁵; 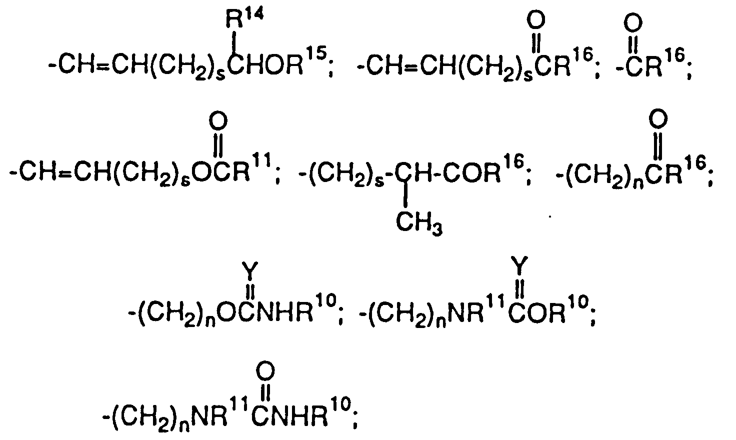 -(CH₂)ₙNR¹¹SO₂R¹⁰; 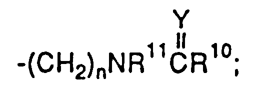 -(CH₂)ₘF; -(CH₂)ₘONO₂; -CH₂N_{3;} -(CH₂)ₘNO₂; -CH=N-NR¹¹R¹⁷; 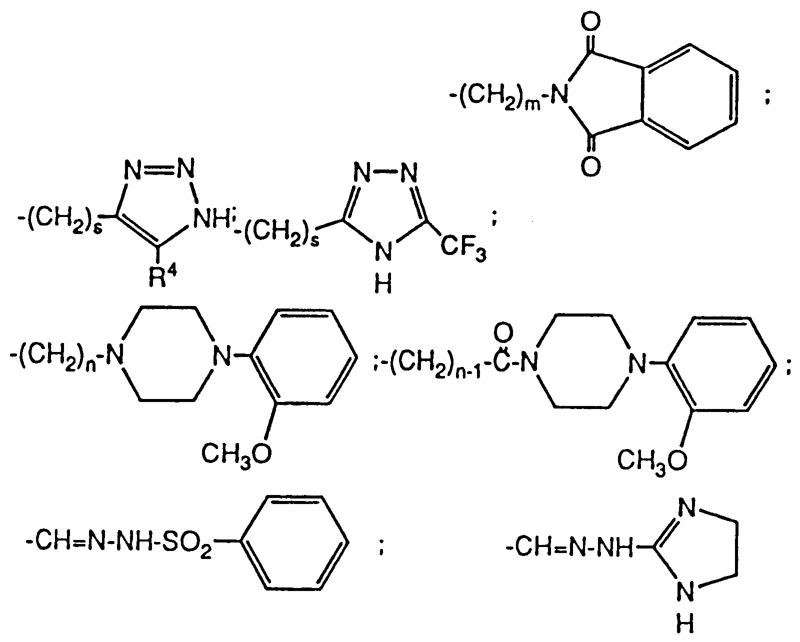 ist; R⁹ 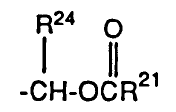 ist;
R¹⁰ ein Alkyl mit 1 bis 6 Kohlenstoffatomen oder ein Perfluoralkyl mit 1 bis 6 Kohlenstoffatomen, 1-Adamantyl, 1-Naphthyl, 1-(1-Naphthyl) ethyl oder (CH₂)ₚC₆H₅ ist;
R¹¹ H, ein Alkyl mit 1 bis 6 Kohlenstoffatomen, ein Cycloalkyl mit 3 bis 6 Kohlenstoffatomen, Phenyl oder Benzyl ist;
R¹² H, Methyl oder Benzyl ist;
R¹³ -CO₂H; -CO₂R⁹; -CH₂CO₂H, -CH₂CO₂R⁹; 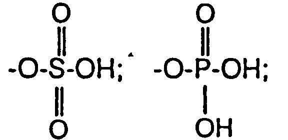 -SO₃H; 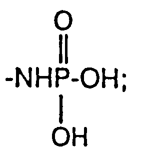 -PO₃H₂; -C(CF₃)₂OH; -NHSO₂CH₃; -NHSO₂CF₃; -NHCOCF₃; -CONHOR¹²; -SO₂NH₂; 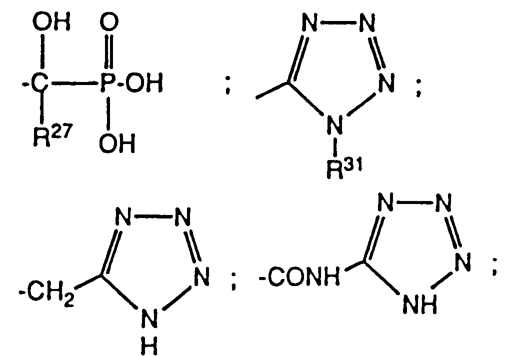 -CONHNHSO₂CF₃ ; 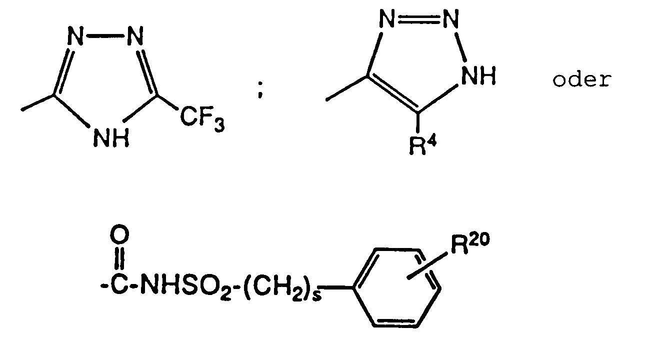 ist;
R¹⁴ H, Alkyl oder ein Perfluoralkyl mit 1 bis 8 Kohlenstoffatomen, ein Cycloalkyl mit 3 bis 6 Kohlenstoffatomen, Phenyl oder Benzyl ist;
R¹⁵ H, ein Alkyl mit 1 bis 6 Kohlenstoffatomen, ein Cycloalkyl mit 3 bis 6 Kohlenstoffatomen, Phenyl, Benzyl, ein Acyl mit 1 bis 4 Kohlenstoffatomen, Phenacyl ist;
R¹⁶ H, ein Alkyl mit 1 bis 6 Kohlenstoffatomen, ein Cycloalkyl mit 3 bis 6 Kohlenstoffatomen, (CH₂)ₚC₆H₅, OR¹⁷ oder NR¹⁸R¹⁹ ist;
R¹⁷ H, ein Alkyl mit 1 bis 6 Kohlenstoffatomen, ein Cycloalkyl mit 3 bis 6 Kohlenstoffatomen, Phenyl oder Benzyl ist;
R¹⁸ und R¹⁹ unabhängig H, ein Alkyl mit 1 bis 4 Kohlenstoffatomen, Phenyl, Benzyl, α-Methylbenzyl sind oder zusammengefaßt mit dem Stickstoff einen Ring der Formel 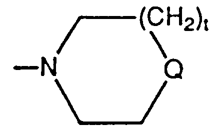 bilden;
Q NR²⁰, O oder CH₂ ist;
R²⁰ H, ein Alkyl mit 1 - 4 Kohlenstoffatomen oder Phenyl ist;
R²¹ ein Alkyl mit 1 bis 6 Kohlenstoffatomen, -NR²²R²³ oder 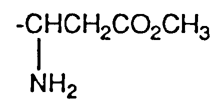 ist;
R²² und R²³ unabhängig voneinander H, ein Alkyl mit 1 bis 6 Kohlenstoffatomen, Benzyl oder als (CH₂)ᵤ, wobei u 3 - 6 ist, zusammengefaßt sind;
R²⁴ H, CH₃ oder -C₆H₅ ist;
R²⁵ NR²⁷R²⁸, OR²⁸, NHCONH₂, NHCSNH₂, 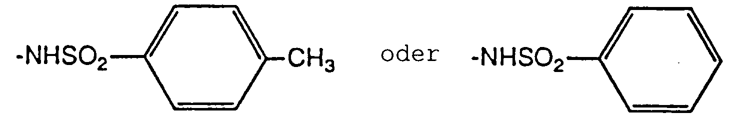 ist;
R²⁶ Wasserstoff, ein Alkyl mit 1 bis 6 Kohlenstoffatomen, Benzyl oder Allyl ist;
R²⁷ und R²⁸ unabhängig voneinander Wasserstoff, ein Alkyl mit 1 bis 5 Kohlenstoffatomen oder Phenyl sind;
R²⁹ und R³⁰ unabhängig voneinander ein Alkyl mit 1 - 4 Kohlenstoffatomen oder zusammengefaßt -(CH₂)_{q}- sind;
R³¹ H, ein Alkyl mit 1 bis 4 Kohlenstoffatomen, -CH₂CH=CH₂ oder -CH₂C₆H₄R³² ist;
R³² H, NO₂, NH₂, OH oder OCH₃ ist;
X eine Kohlenstoff-Kohlenstoff-Einfachbindung, -CO-, -CH₂-, -O-, -S-, -NH-, 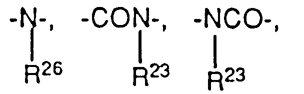 -OCH₂-, -CH₂O-, -SCH₂-, -CH₂S-, -NHC(R²⁷)(R²⁸)-, -NR²³SO₂-, -SO₂NR²³-, -C(R²⁷)(R²⁸)NH-, -CH=CH-, -CF=CF-, -CH=CF-, -CF=CH-, -CH₂CH₂-, -CF₂CF₂-, 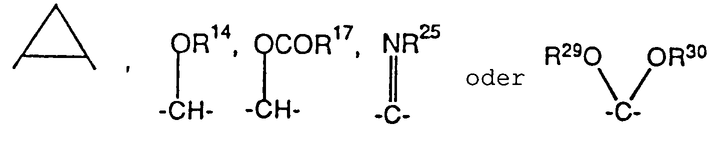 ist;
Y O oder S ist;
Z O, NR¹¹ oder S ist;
m 1 bis 5 ist;
n 1 bis 10 ist;
p 0 bis 3 ist;
q 2 bis 3 ist;
r 0 bis 2 ist;
s 0 bis 5 ist;
t 0 oder 1 ist;
und eines pharmazeutisch annehmbaren Salzes dieser Verbindungen; mit der Maßgabe, daß:
(1) die R¹-Gruppe nicht in der ortho-Position ist;
(2) wenn R¹ 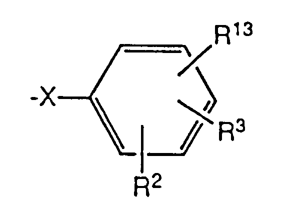 ist, X eine Einfachbindung ist und R¹³ CO₂H oder 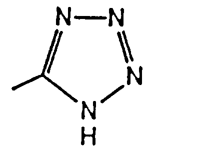 ist, dann R¹³ sich in der ortho- oder meta-Position befinden muß oder, wenn R¹ und X wie oben sind und R¹³ NHSO₂CF₃ oder NHSO₂CH₃ ist, dann R¹³ ortho sein muß;
(3) wenn R¹ 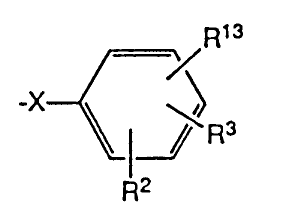 ist und X eine andere als eine Einfachbindung ist, dann R¹³ ortho sein muß, mit der Ausnahme, daß, wenn X = NR²³CO und R¹³ NHSO₂CF₃ oder NHSO₂CH₃ ist, dann R¹³ ortho oder meta sein muß;
(4) wenn R¹ 4-CO₂H oder dessen Salz ist, dann R⁶ nicht S-Alkyl sein kann;
(5) wenn R¹ 4-CO₂H oder dessen Salz ist, der Substituent an der 4-Position des Imidazols nicht CH₂OH, CH₂OCOCH₃ oder CH₂CO₂H sein kann;
(6) wenn R¹ 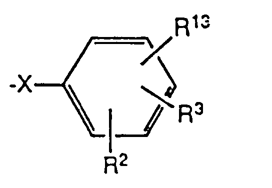 ist, X -OCH₂- ist und R¹³ 2-CO₂H ist und R⁷ H ist, dann R⁶ nicht C₂H₅S ist;
(7) wenn R¹ 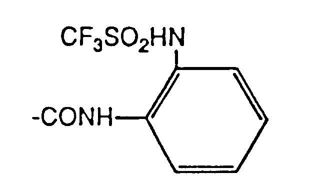 ist und R⁶ n-Hexyl ist, dann R⁷ und R⁸ nicht beide Wasserstoff sind;
(8) wenn R¹ 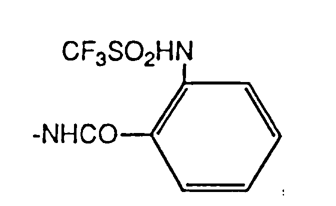 ist, dann R⁶ nicht Methoxybenzyl ist;
(9) die R⁶-Gruppe nicht -CHFCH₂CH₂CH₃ oder CH₂OH ist;
(10) wenn r = 0, R¹ 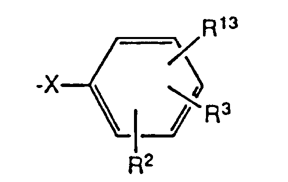 ist, X -NH-CO- ist, R¹³ 2-NHSO₂CF₃ ist und R⁶ n-Propyl ist, dann R⁷ und R⁸ nicht -CO₂CH₃ sind;
(11) wenn r = 0, R¹ 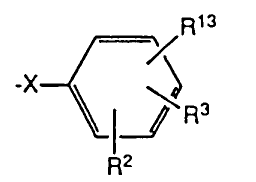 ist, X -NH-CO- ist, R¹³ 2-COOH ist und R⁶ n-Propyl ist, dann R⁷ und R⁸ nicht -CO₂CH₃ sind;
(12) wenn r = 1, R¹ 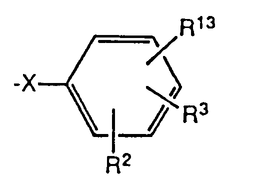 ist, X eine Einfachbindung ist, R⁷ Cl ist und R⁸ - -CHO ist, dann R¹³ nicht 3-(Tetrazol-5-yl) ist;
(13) wenn r = 1, R¹ 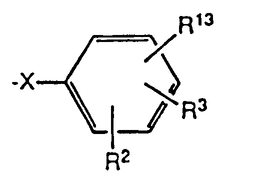 ist, X eine Einfachbindung ist, R⁷ Cl ist und R⁸ -CHO ist, dann R¹³ nicht 4-(Tetrazol-5-yl) ist,
zur Herstellung eines Medikaments zur Behandlung von chronischem, durch Angiotensin bewirktem Nierenversagen.

2. Verwendung nach Anspruch 1, wobei die Verbindung der Formel I eine Verbindung der Formel II: 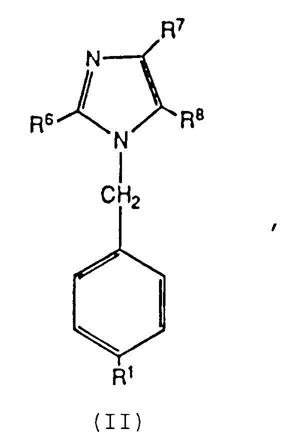 wobei R¹ -CO₂H; -NHSO₂CF₃; 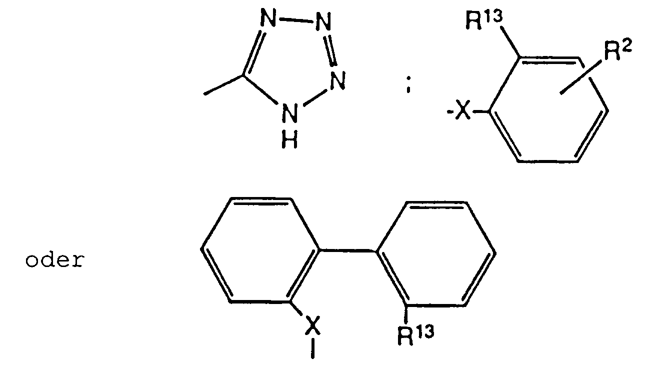 ist;
R⁶ ein Alkyl mit 3 bis 10 Kohlenstoffatomen, ein Alkenyl mit 3 bis 10 Kohlenstoffatomen, ein Alkinyl mit 3 bis 10 Kohlenstoffatomen, ein Cycloalkyl mit 3 bis 8 Kohlenstoffatomen, ein Benzyl, das am Phenylring mit bis zu zwei Gruppen, ausgewählt aus einem Alkoxy mit 1 bis 4 Kohlenstoffatomen, einem Halogen, einem Alkyl mit 1 bis 4 Kohlenstoffatomen und Nitro substituiert ist;
R⁸ -(CH₂)ₛ-Tetrazolyl, -(CH₂)ₙOR¹¹; 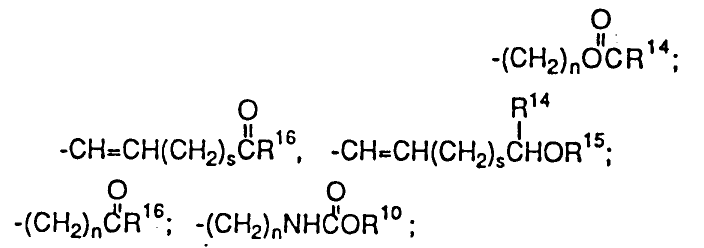 -(CH₂)ₙNHSO₂R¹⁰; -(CH₂)ₘF oder 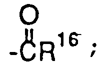 ein Phenylalkenyl, wobei der aliphatische Teil 2 bis 4 Kohlenstoffatome umfaßt; ein -(CH₂)ₘ-imidazol-1-yl oder ein -(CH₂)ₘ-1,2,3-Triazolyl ist, das gegebenenfalls durch eine oder zwei Gruppen, ausgewählt aus -CO₂CH₃ oder ein Alkyl mit 1 bis 4 Kohlenstoffatomen, substituiert ist;
R¹³ -CO₂H, -CO₂R⁹, NHSO₂CF₃; SO₃H oder 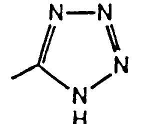 ist;
R¹⁶ H, ein Alkyl mit 1 bis 5 Kohlenstoffatomen, OR¹⁷ oder NR¹⁸R¹⁹ ist;
X eine Kohlenstoff-Kohlenstoff-Einfachbindung, -CO-, -CH₂CH₂-, 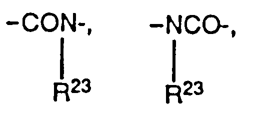 -OCH₂-, -CH₂O, -O-, SCH₂-, -CH₂S-, -NH-CH₂-, -CH₂NH- oder -CH=CH- ist; oder deren pharmazeutisch annehmbares Salz ist.

3. Verwendung nach Anspruch 2, wobei die Verbindung der Formel II eine Verbindung, in der:
R² H, ein Alkyl mit 1 bis 4 Kohlenstoffatomen, ein Halogen oder ein Alkoxy mit 1 bis 4 Kohlenstoffatomen ist;
R⁶ ein Alkyl, ein Alkenyl oder Alkinyl mit 3 bis 7 Kohlenstoffatomen ist;
R⁷ ein aus 2- und 3-Thienyl, 2- und 3-Furyl, 2-, 3- und 4-Pyridyl ausgewähltes Heteroaryl, p-Biphenylyl; H, Cl, Br, I; CᵥF₂ᵥ₊₁, wobei v = 1 - 3; 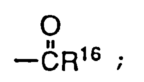 einem grad- oder verzweigtkettigen Alkyl mit 1 bis 6 Kohlenstoffatomen oder Phenyl ist;
R⁸ -(CH₂)ₘOR¹¹; 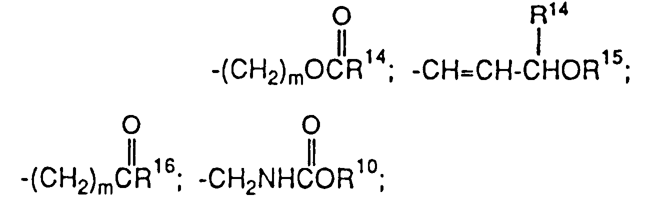 -(CH₂)ₘNHSO₂R¹⁰; 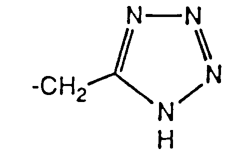 oder -COR¹⁶ ist,
R¹⁰ CF₃, ein Alkyl mit 1 bis 6 Kohlenstoffatomen oder Phenyl ist;
R¹¹ H oder ein Alkyl mit 1 bis 4 Kohlenstoffatomen ist;
R¹³ CO₂H; CO₂CH₂OCOC(CH₃)₃; NHSO₂CF₃; 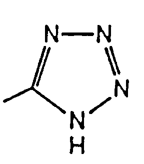
ist;
R¹⁴ H oder ein Alkyl mit 1 bis 4 Kohlenstoffatomen ist;
R¹⁵ H, ein Alkyl mit 1 bis 4 Kohlenstoffatomen oder ein Acyl mit 1 bis 4 Kohlenstoffatomen ist;
R¹⁶ H, ein Alkyl mit 1 bis 5 Kohlenstoffatomen; OR¹⁷ oder 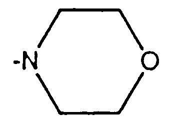 ist;
m 1 bis 5 ist;
X = Einfachbindung, -O-; -CO-; -NHCO oder -OCH₂-, oder deren pharmazeutisch annehmbares Salz ist.

4. Verwendung nach Anspruch 3, wobei die Verbindung der Formel II eine Verbindung, worin R¹ 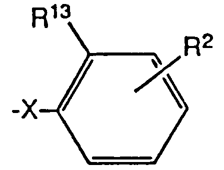 ist und X eine Einfachbindung ist; oder deren pharmazeutisch annehmbares Salz ist.

5. Verwendung nach Anspruch 4, wobei die Verbindung der Formel II 2-Butyl-4-chlor-1-[(2'-(lH-tetrazol-5-yl)biphenyl-4-yl)methyl]-5-(hydroxymethyl)imidazol, oder deren pharmazeutisch annehmbares Salz ist.

6. Verwendung nach Anspruch 4, wobei die Verbindung der Formel II 2-Butyl-4-chlor-1-[(2'-carboxybiphenyl-4-yl)methyl]-5-(hydroxymethyl)imidazol, oder deren pharmazeutisch annehmbares Salz ist.

7. Verwendung nach Anspruch 4, wobei die Verbindung der Formel II 2-Butyl-4-chlor-1-[(2'-carboxybiphenyl-4-yl)methyl]-5-[(methoxycarbonyl)aminomethyl]imidazol, oder deren pharmazeutisch annehmbares Salz ist.

8. Verwendung nach Anspruch 4, wobei die Verbindung der Formel II 2-Butyl-4-chlor-1-[(2'-carboxybiphenyl-4-yl)methyl]-5-[(propoxycarbonyl)aminomethyl]imidazol, oder deren pharmazeutisch annehmbares Salz ist.

9. Verwendung nach Anspruch 4, wobei die Verbindung der Formel II 2-Butyl-4-chlor-1-[(2'-carboxybiphenyl-4-yl)methyl]imidazol-5-carboxaldehyd, oder deren pharmazeutisch annehmbares Salz ist.

10. Verwendung nach Anspruch 4, wobei die Verbindung der Formel II 2-Butyl-1-[(2'-carboxybiphenyl-4-yl)methyl]imidazol-5-carboxaldehyd, oder deren pharmazeutisch annehmbares Salz ist.

11. Verwendung nach Anspruch 4, wobei die Verbindung der Formel II 2-(1E-Butenyl)-4-chlor-1-[(2'-carboxybiphenyl-4-yl)methyl]-5-(hydroxymethyl)imidazol, oder deren pharmazeutisch annehmbares Salz ist.

12. Verwendung nach Anspruch 4, wobei die Verbindung der Formel II 2-(lE-Butenyl)-4-chlor-1-[(2'-carboxybiphenyl-4-yl)methyl]imidazol-5-carboxaldehyd, oder deren pharmazeutisch annehmbares Salz ist.

13. Verwendung nach Anspruch 4, wobei die Verbindung der Formel II 2-Propyl-4-chlor-1-[(2'-(lH-tetrazol-5-yl)biphenyl-4-yl)methyl]-5-(hydroxymethyl)imidazol, oder deren pharmazeutisch annehmbares Salz ist.

14. Verwendung nach Anspruch 4, wobei die Verbindung der Formel II 2-Propyl-4-chlor-1-[(2'-(lH-tetrazol-5-yl)biphenyl-4-yl)methyl]imidazol-5-carboxaldehyd, oder deren pharmazeutisch annehmbares Salz ist.

15. Verwendung nach Anspruch 4, wobei die Verbindung der Formel II 2-Butyl-4-chlor-l-[2'-(lH-tetrazol-5-yl)biphenyl-4-yl)methyl]imidazol-5-carboxaldehyd, oder deren pharmazeutisch annehmbares Salz ist.

16. Verwendung nach Anspruch 4, wobei die Verbindung der Formel II 2-[1E-Butenyl)-4-chlor-1-[(2'-(1H-tetrazol-5-yl)biphenyl-4-yl)methyl]-5-hydroxymethyl)imidazol, oder deren pharmazeutisch annehmbares Salz ist.

17. Verwendung nach Anspruch 4, wobei die Verbindung der Formel II 2-(1E-Butenyl)-4-chlor-1-[(2'-(lH-tetrazol-5-yl)biphenyl-4-yl)methyl]imidazol-5-carboxaldehyd, oder deren pharmazeutisch annehmbares Salz ist.

18. Verwendung nach Anspruch 4, wobei die Verbindung der Formel II 2-Butyl-4-chlor-1-[(2'-(1H-tetrazol-5yl)-biphenyl-4-yl)methyl]imidazol-5-carbonsäure, oder deren pharmazeutisch annehmbares Salz ist.

19. Verwendung nach Anspruch 4, wobei die Verbindung der Formel II 2-Propyl-4-chlor-1-[(2'-(1H-tetrazol-5-yl)-biphenyl-4-yl)methyl]imidazol-5-carbonsäure, oder deren pharmazeutisch annehmbares Salz ist.

20. Verwendung nach Anspruch 4, wobei die Verbindung der Formel II 2-Propyl-4-trifluormethyl-1-[(2'-(1H-tetrazol-5-yl)biphenyl-4-yl)methyl]imidazol-5-carbonsäure, oder deren pharmazeutisch annehmbares Salz ist.

21. Verwendung nach Anspruch 4, wobei die Verbindung der Formel II 2-Propyl-4-trifluormethyl-1-[(2'-(lH-tetrazol-5-yl)biphenyl-4-yl)methyl]-S-(hydroxylmethyl)imidazol, oder deren pharmazeutisch annehmbares Salz ist.

22. Verwendung nach Anspruch 4, wobei die Verbindung der Formel II 2-Butyl-4-trifluormethyl-1-[(2'-(1H-tetrazol-5-yl)biphenyl-4-yl)methyl]imidazol-5-carbonsäure, oder deren pharmazeutisch annehmbares Salz ist.

23. Verwendung nach Anspruch 4, wobei die Verbindung der Formel II 2-Propyl-4-trifluormethyl-1-[(2'-(carboxybiphenyl-4-yl)methyl]imidazol-5-carboxaldehyd, oder deren pharmazeutisch annehmbares Salz ist.

24. Verwendung nach Anspruch 4, wobei die Verbindung der Formel II 2-Propyl-4-pentafluorethyl-1-[(2'-(1H-tetrazol-5-yl)biphenyl-4-yl)methyl]-5-(hydroxymethyl)imidazol, oder deren pharmazeutisch annehmbares Salz ist.

25. Verwendung nach Anspruch 4, wobei die Verbindung der Formel II 2-Propyl-4-pentafluorethyl-1-[(2'-(1H-tetrazol-5-yl)biphenyl-4-yl)methyl]imidazol-5-carbonsäure, oder deren pharmazeutisch annehmbares Salz ist.

26. Verwendung nach Anspruch 4, wobei die Verbindung der Formel II 2-Propyl-4-pentafluorethyl-[(2'-(1H-tetrazol-5-yl)biphenyl-4-yl)methyl]imidazol-5-carboxaldehyd, oder deren pharmazeutisch annehmbares Salz ist.

27. Verwendung nach Anspruch 4, wobei die Verbindung der Formel II 1-[(2'-(1H-Tetrazol-5-yl)biphenyl-4-yl)methyl]-4-phenyl-2-propylimidazol-5-carboxaldehyd, oder deren pharmazeutisch annehmbares Salz ist.

28. Verwendung nach Anspruch 4, wobei die Verbindung der Formel II 1-[(2'-Carboxybiphenyl-4-yl)methyl]-4-phenyl-2-propylimidazol-5-carboxaldehyd, oder deren pharmazeutisch annehmbares Salz ist.

## Revendications

1. Utilisation d'un composé de formule (I): 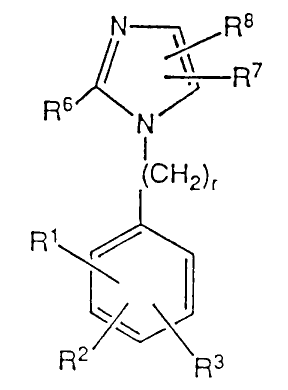 dans laquelle:
R¹ représente 4-CO₂H; 4-CO₂R⁹; 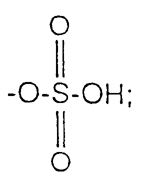 -SO₃H; -C(CF₃)₂OH; 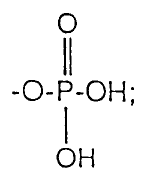 -PO₃H₂; 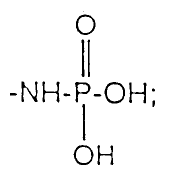 4-NHSO₂CH₃; -4-NHSO₂CF₃; -CONHOR¹²; -SO₂NH₂; 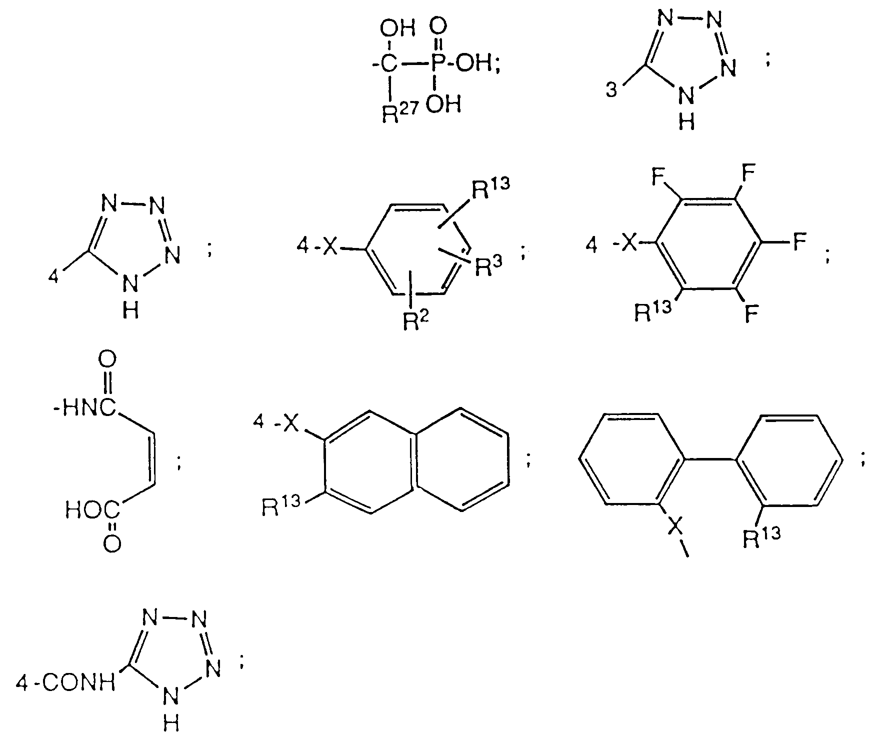 4-CONHNHSO₂CF₃; 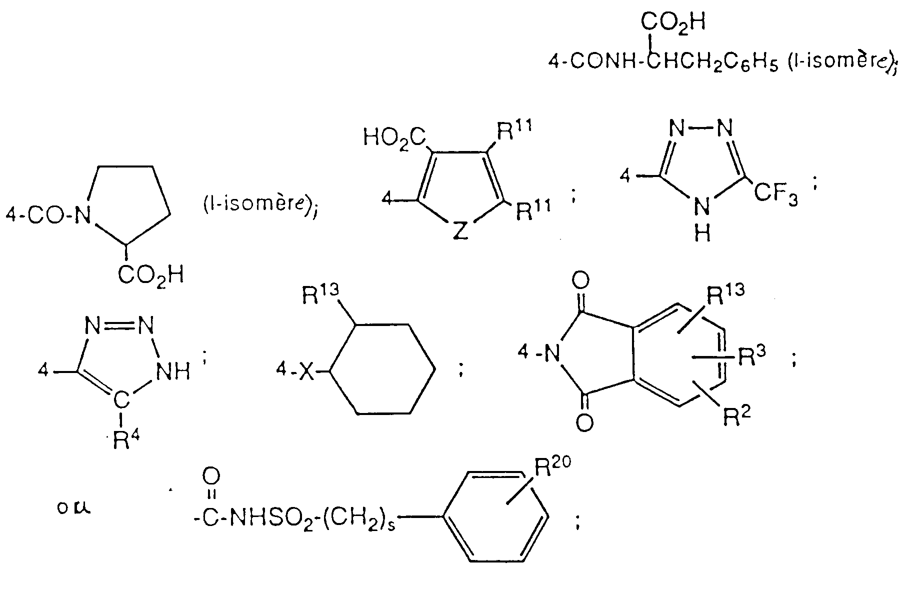
R² représente H; Cl; Br; I; F; NO₂; CN; alkyle en C₁-C₄; acyloxy en C₁-C₄; alkoxy en C₁-C₄; CO₂H; CO₂R⁹; 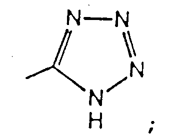 NHSO₂CH₃; NHSO₂CF_{3;} CONHOR¹²; SO₂NH₂; aryle; ou furyle;
R³ représente H; Cl, Br, I ou F; alkyle en C₁-C₄ ou alkoxy en C₁-C₄;
R⁴ représente CN, NO₂ ou CO₂R¹¹;
R⁵ représente H, alkyle en C₁-C₆, cycloalkyle en C₃-C₆, alkényle ou alkynyle en C₂-C₄;
R⁶ représente un alkyle en C₂-C₁₀, alkényle ou alkynyle en C₃-C₁₀ ou les mêmes groupes substitués par F ou CO₂R¹⁴; cycloalkyle en C₃-C₈, cycloalkylalkyle en C₄-C₁₀; cycloalkylalkényle ou cycloalkylalkynyle en C₅-C₁₀; (CH₂)ₛZ(CH₂)ₘR⁵ éventuellement substitué par F Ou CO₂R¹⁴; benzyle ou benzyle substitué sur le cycle phényle par 1 ou 2 halogènes, alkoxy en C₁-C₄, alkyle en C₁-C₄ ou nitro;
R⁷ représente H, F, Cl, Br, I, NO₂, CᵥF₂ᵥ, où v=1-6; C₆F₅; CN; ;
alkyle en C₁-C₆ linéaire ou ramifié; phényle ou phénylalkyle où le radical alkyle comporte 1 à 3 atomes de carbone; ou phényle substitué ou phénylalkyle substitué, où le radical alkyle comporte 1 à 3 atomes de carbone, substitués par 1 ou 2 substituants sélectionnés parmi alkyle en C₁-C₄, F, Cl, Br, OH, OOCH₃, CF₃, et COOR, où R représente H, alkyle en C1-C₄, ou phényle; vinyle; alkynyle en C₁-C₁₀; phénylalkynyle où la partie alkynyle comporte 2 à 6 atomes de carbone; hétéroaryl sélectionné parmi 2- et 3-thiényl, 2- et 3-furyle, 2-, 3-, et 4-pyridyle, 2-pyrazinyle, 2-, 4- et 5-pyrimidinyle, 3- et 4-pyridazinyle, 2-, 4- et 5-thiazolyle, 2-, 4- et 5-sélénazolyle, 2- 4-, et 5-oxazolyle; 2- ou 3- pyrrolyle, 3-, 4- ou 5-pyrazolyle, et 2-, 4- ou 5-imidazolyle; o-, m- ou p-biphénylyle; o-, m- ou p-phénoxyphényle; phénylalkynyle, hétéroaryle, biphényle ou phénoxyphényle substitués tels que définis ci-dessus, substitués sur les carbones du cycle par un ou deux substituants sélectionnés parmi halogène, alkoxy en C1-C5, alkyle en C₁-C₅, -NO₂, -CN, -CF₃, -COR¹⁶, -CH₂OR¹⁷, - NHCOR¹⁷, CONR¹⁸R¹⁹, S(O)ₙR¹⁷, et SO₂NR¹⁸R¹⁹; pyrrolyle, pyrazolyle ou imidazolyle, tels que définis ci-dessus, substitués sur l'atome d'azote du cycle par un alkyle en C₁-C₅ ou un benzyle; ou alkyle, alkényle ou alkynyle comportant 1 à 10 atomes de carbone substitués par un groupe hétéroaryle, biphénylyle ou phénoxyphényle tel que défini ci-dessus; tous les groupes aryles polycycliques ci-dessus substitués par 1 ou 2 substituants sélectionnés parmi halogène, alkoxy en C₁-C₅, allyle en C₁-C₅, -NO₂, -CN, -CF₃, -COR¹⁶, -CH₂OR¹⁷, -NHCOR¹⁷, CONRR¹⁹, S(O)ₙR¹⁷, et SO₂NR¹⁸R¹⁹; l'anhydride du 4,5-dicarboxyl-1- ou 2-naphtyle; ou alkyle en C₁-C₁₀ substitué, alkényle ou alkynyle en C₂-C₁₀ substitué par un groupe aryle, polycyclique substitué ou non substitué tel que défini ci-dessus.
R⁸ représente H, CN, alkyle en C₁-C₁₀, alkényle en C₃-C₁₀, ou les mêmes groupes substitués par F; phénylalkényle où la partie aliphatique comporte 2 à 6 atomes de carbone; -(CH₂)ₘ-imidazol-1-yle; -(CH₂)ₘ-1,2,3-triazolyle éventuellement substitués par un ou deux groupes sélectionnés parmi CO₂CH₃ ou alkyle en C₁-C₄; -(CH₂)ₛ-tétrazolyle; 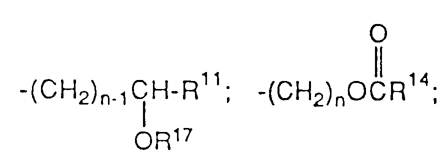 -(CH₂)ₙSR¹⁵; 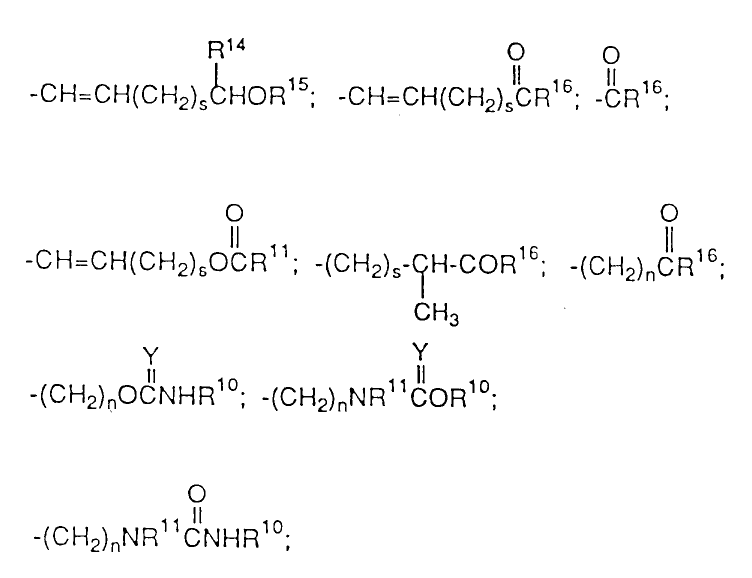 -(CH₂)ₙNR¹¹SO₂R¹⁰; 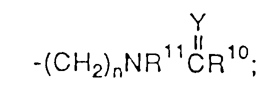 -(CH₂)ₘF; -(CH₂)ₘONO₂; -CH₂N_{3;} -(CH₂)ₘNO₂; -CH=N-NR¹¹R¹⁷; 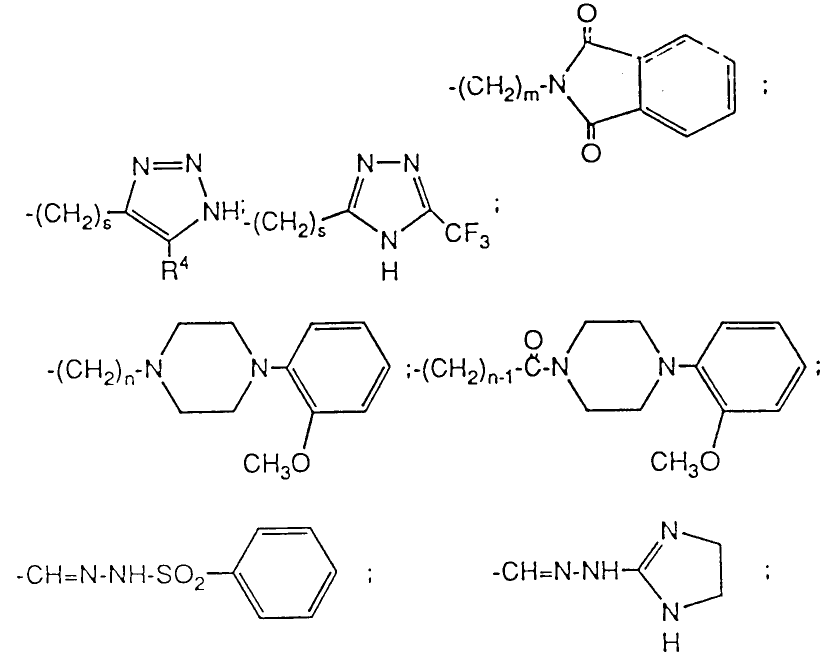
R⁹ représente ; 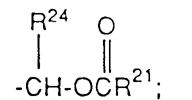
R¹⁰ représente un alkyle en C₁-C₆ ou perfluoroalkyle en C₁-C₆, 1-adamantyle, 1-naphtyle, 1-(1-naphtyl)éthyle, ou (CH₂)ₚC₆H₅;
R¹¹ représente H, alkyle en C₁-C₆, cycloalkyle en C₃-C₆, phényle ou benzyle;
R₁₂ représente H, méthyle ou benzyle;
R₁₃ représente -CO₂H; -CO₂R⁹; -CH₂CO₂H, CH₂CO₂R⁹;
-CO₂H; -CO₂R⁹; -CH₂CO₂H, -CH₂CO₂R⁹; 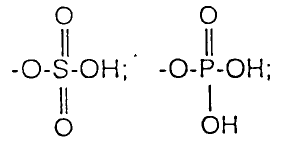 -SO₃H; 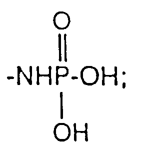 -PO₃H₂; -C(CF₃=)₂OH; -NHSO₂CH₃; -NHSO₂CF₃; -NHCOCF₃; -CONHOR¹²; -SO₂NH₂; 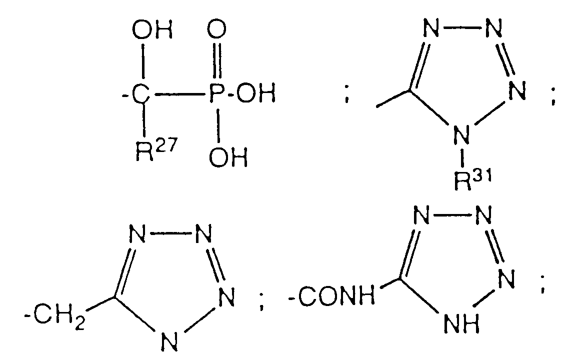 -CONHNHSO₂CF₃ 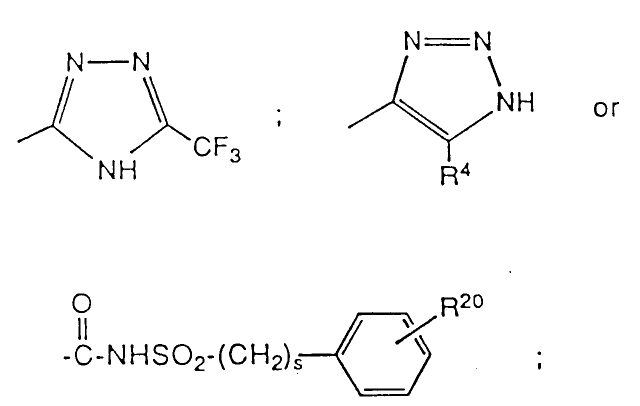
R₁₄ représente H, alkyle ou perfluoroalkyle en C₁-C₈, cycloalkyle en C₃-C₆, phényle ou benzyle;
R¹⁵ représente H, alkyle en C₁-C₆, cycloalkyle en C₃-C₆, phényle, benzyle, acyle en C₁-C₄, phénacyle;
R¹⁶ représente H, alkyle en C₁-C₆, cycloalkyle en C₃-C₆, (CH₂)C₆H₅, OR¹⁷, ou NR¹⁸R¹⁹;
R¹⁷ représente H, alkyle en C₁-C₆, cycloalkyle en C₃-C₆; phényle ou benzyle;
R¹⁸ et R¹⁹ représentent indépendamment l'un de l'autre H, alkyle en C₁-C₄, phényle, benzyle, α-méthylbenzyle, ou forment mensemble avec l'atome d'azote un cycle de formule: 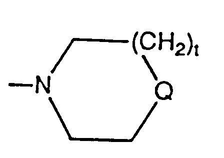
Q représente NR²⁰, O ou CH₂;
R²⁰ représente H, alkyle en C₁-C₄, ou phényle;
R²¹ représente alkyle en C₁-C₆, -NR²²R²³, ou 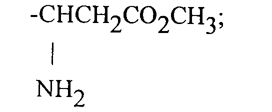
R²² et R²³ représentent indépendamment l'un de l'autre H, alkyle en C₁-C₆, benzyle, ou forment ensemble (CH₂)ᵤ où u est 3-6.
R²⁴ représente H, CH₃ ou -C₆H₅;
R²⁵ représente NR²⁷R²⁸, OR²⁸, NHCONH₂, NHCSNH₂; 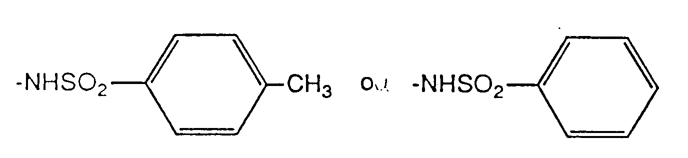
R²⁶ représente H, alkyle en C₁-C₆, benzyle ou allyle;
R²⁷ et R²⁸ représentent indépendamment l'un de l'autre H, alkyle en C₁-C₅, ou phényle;
R²⁹ et R³⁰ représentent indépendamment l'un de l'autre alkyle en C₁-C₄ ou forment ensemble -(CH₂)_{q}-;
R³¹ représente H, alkyle en C₁-C₄, -CH₂CH=CH₂ ou -CH₂C₆H₄R³²
R³² représente H, NO₂, NH₂, OH ou OCH₃;
X représente une liaison C-C simple, -CO-, -CH₂-, -O-, -S-, -NH-, 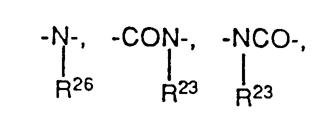 -OCH₂-, -CH₂O-, -SCH₂-, -CH₂S-, -NHC(R²⁷)(R²⁸)-, -NR²³SO₂-, -SO₂NR²³-, -C(R²⁷)(R²⁸)NH-, -CH=CH-, -CF=CF-, -CH=CF-, -CF=CH-, -CH₂CH₂-, -CF₂CF₂-, 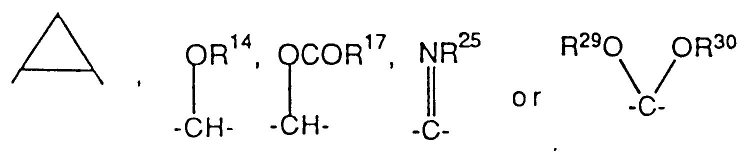
Y représente O ou S;
Z représente O, NR¹¹, ou S;
m représente 1 à 5.
n représente 1 à 10;
p représente 0 à 3;
q représente 2 à 3;
r représente 0 à 2;
s représente 0 à 5;
t représente 0 ou 1;
ou un sel pharmaceutiquement acceptable en dérivant; à condition que:
(1) le groupe R¹ ne soit pas en position ortho;
(2) lorsque R¹ représente 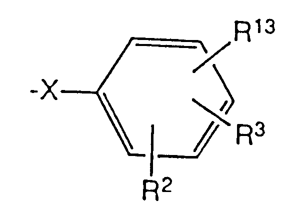
X est une liaison simple, et R¹³ représente CO₂H, ou 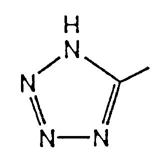
R¹³ doit être en position ortho ou méta; ou lorsque
R¹ et X sont tels que définis ci-dessus et R₁₃ est NHSO₂CF₃ oui NHSO₂CH₃,
R¹³ doit être en position ortho;
(3) lorsque R¹ représente: 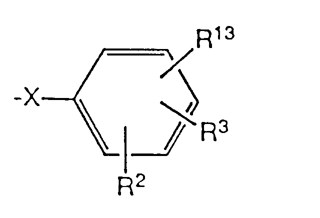
et X ne représente pas une liaison simple, alors R¹³ doit être en position ortho, sauf lorsque X représente NR²³CO et R¹³ représente NHSO₂CF₃ ou NHSO₂CH₃, alors R¹³ doit être en position ortho ou meta;
(4) lorsque R¹ représente 4-CO₂H ou un sel en dérivant, R⁶ ne doit pas représenter S-alkyle;
(5) lorsque R¹ représente 4-CO₂H ou un sel en dérivant, le substituant en position 4 de l'imidazole ne peut pas être CH₂OH, CH₂OCOCH₃, ou CH₂CO₂H;
(6) lorsque R¹ représente 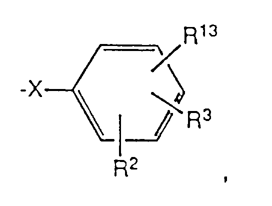
X représente -OCH₂-, et R¹³ représente 2-CO₂H, et R⁷ représente H alors R⁶ ne représente pas C₂H₅S;
(7) lorsque R¹ représente: 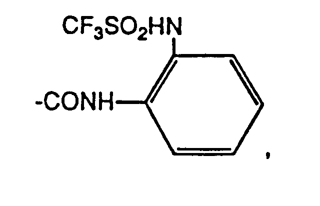
et R⁶ est le n-hexyle, alors R⁷ et R⁸ ne représentent pas tous deux un atome hydrogène;
(8) lorsque R¹ représente: 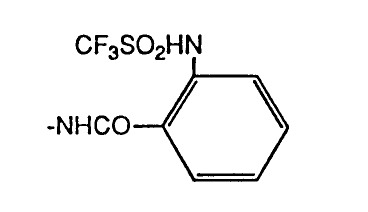
R⁶ ne représente pas un groupe méthoxybenzyle;
(9) le groupe R⁶ n'est pas -CHFCH₂CH₂CH₃ ou CH₂OH;
(10) lorsque r=0, R¹ représente: 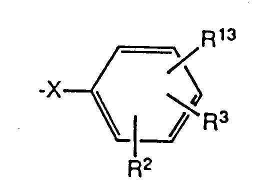
X représente-NH-CO-, R¹³ représente 2-NHSO₃CF₃; et R⁶ représente n-propyle, alors R⁷ et R⁸ ne sont pas -CO₂CH₃;
(11) lorsque r=0, R¹ représente: 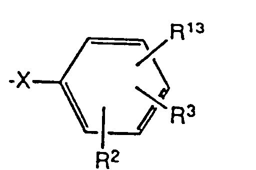
X représente -NH-CO-, R¹³ représente 2-COOH, et R⁶ représente n-propyle, alors R⁷ et R⁸ ne sont pas -CO₂CH₃;
(12) lorsque r=1, R¹= 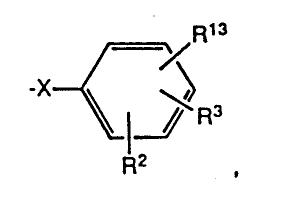
X est une liaison simple, R⁷ représente Cl, et R⁸ représente -CHO, alors R¹³ n'est pas 3-(tétrazol-5-yle);
(13) lorsque r=1, R¹= 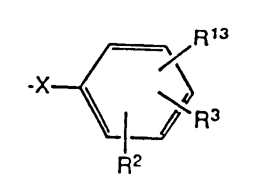
X est une liaison simple, R⁷ représente Cl, et R⁸ est -CHO, alors R¹3 n'est pas 4-(tétrazol-5-yle) pour la préparation d'un médicament pour le traitement de l'insuffisance rénale chronique due à l'angiotensine II.

2. Utilisation selon la revendication 1, dans laquelle le composé de formule I correspond à un composé de formule II: 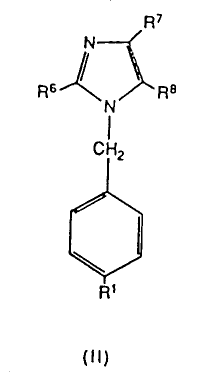
R¹ représente -CO₂H; -NHSO₂CF₃; 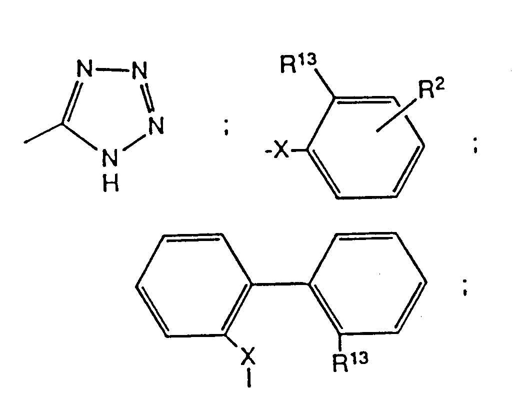 ou
R⁶ représente un alkyle en C₃-C₁₀, alkényle en C₃-C₁₀, alkynyle en C₃-C₁₀, cycloalkyle en C₃-C₈, benzyle substitué sur le cycle phényle par jusqu'à deux groupes sélectionnés parmi alkoxy en C₁-C₄, atomes d'halogène, alkyle en C₁-C₄, et nitro;
R⁸ représente: -(CH₂)ₛ-tetrazolyl, -(CH₂)ₙOR¹¹; 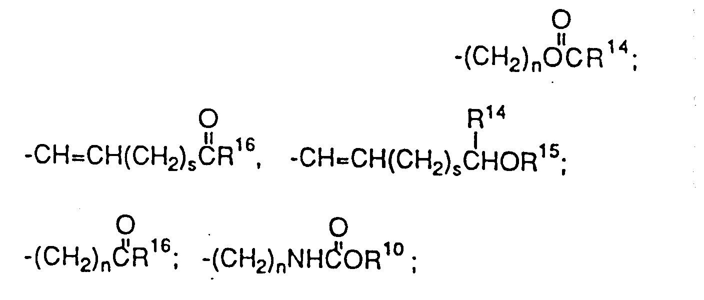 -(CH₂)ₙNHSO₂R¹⁰; -(CH₂)ₘF; or 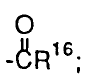 -(CH₂)ₛ-tetrazolyl, -(CH₂)ₙOR¹¹; 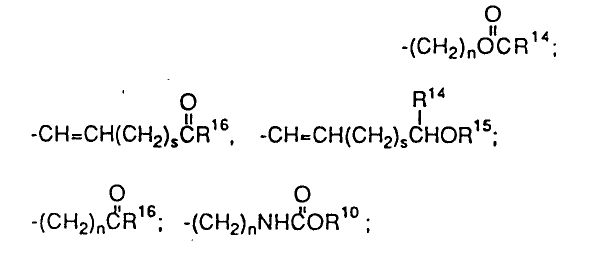 -(CH₂)ₙNHSO₂R¹⁰; -(CH₂)ₘF; ou 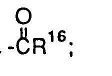 phénylalkényle dans lequel la partie aliphatique comporte 2 à 4 atomes de carbone; -(CH₂)m-imidazol-1-yle; ou -(CH₂)m-1,2,3-triazolyle éventuellement substitué par un à deux groupes sélectionnés parmi-CO₂CH₃ ou alkyle en C₁-C₄;
R¹³ représente -CO₂H, -CO₂R⁹, NHSO₂CF₃; SO₃H; ou 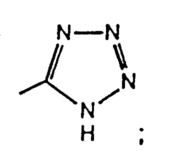
R¹⁶ représente H, alkyle en C₁-C₅, OR¹⁷ ou NR¹⁸R¹⁹;
X représente une liaison simple de carbone-carbone, -CO-, -CH₂CH₂-, 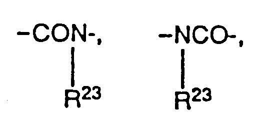 -OCH₂-, -CH₂O-, -O-, -SCH₂-, -CH₂S-, -NH-CH₂-, -CH₂NH- ou -CH=CH-; ou un sel pharmaceutiquement acceptable en dérivant.

3. L'utilisation selon la revendication 2, dans laquelle le composé de formule II est un composé dans lequel:
R² représente H, alkyle en C₁-C₄, halogène ou alkoxy en C₁-C₄;
R⁶ représente alkyle, alkényle ou alkynyle en C₃-C₇;
R⁷ représente un hétéroaryle sélectionné parmi 2- et 3-thiényle, 2- et 3-furyle, 2-, 3-, et 4-pyridyle, p-biphényle; H, Cl, Br, I; CᵥF₂ᵥ₊₁, où v=1-3; 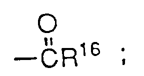 alkyle en C₁-C₆, linéaire ou ramifié; ou phényle;
R⁸ représente: 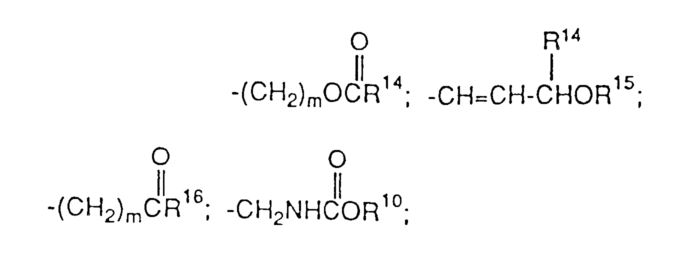 -(CH₂)ₘOR¹¹; -(CH₂)ₘNHSO₂R¹⁰; 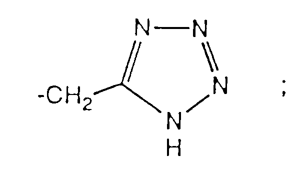 ou -COR¹⁶;
R¹⁰ représente CF₃, alkyle en C₁-C₆ ou phényle;
R¹¹ représente H, ou alkyle en C₁-C₄;
R¹³ représente CO₂H; CO₂CH₂OCOC(CH₃)₃; NHSO₂CF₃; 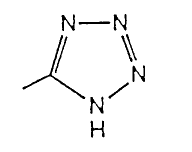
R¹⁴ représente H, ou alkyle en C₁-C₄;
R¹⁵ représente HM, alkyle en C₁-C₄, ou acyle en C₁-C₄;
R¹⁶ représente H, alkyle en C₁-C₅; OR¹⁷; ou 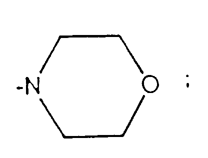
m représente 1 à 5;
X = liaison simple, -O-; -CO-; -NHCO-; ou -OCH₂-; ou un sel pharmaceutiquement acceptable en dérivant.

4. L'utilisation selon la revendication 3, dans laquelle le composé de formule II est un composé dans lequel R¹ représente: 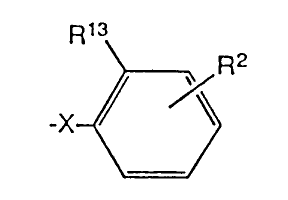 et
X est une liaison simple; ou un sel pharmaceutiquement acceptable en dérivant.

5. L'utilisation selon la revendication 4, dans laquelle le composé de formule II représente le 2-butyl-4-chloro-1-[(2'-(1H-tétrazol-5-yl)biphényl-4-yl)méthyl]-5-(hydroxyméthyl)imidazole ou un sel pharmaceutiquement acceptable en dérivant.

6. L'utilisation selon la revendication 4, dans laquelle le composé de formule II représente le 2-butyl-4-chloro-1-[(2'-carboxyxbiphényl-4-yl)-méthyl]-5-(hydroxyméthyl)imidazole ou un sel pharmaceutiquement acceptable en dérivant.

7. L'utilisation selon la revendication 4, dans laquelle le composé de formule II représente le 2-butyl-4-chloro-1-[(2'-carboxyphényl-4-yl)-méthyl]-5-[(méthoxycarbonyl)aminométhyl]imidazole ou un sel pharmaceutiquement acceptable en dérivant.

8. L'utilisation selon la revendication 4, dans laquelle le composé de formule II représente le 2-butyl-4-chloro-1-[(2'-carboxybiphényl-4-yl)-méthyl]-5-[(propoxycarbonyl)aminométhyl]imidazole ou un sel pharmaceutiquement acceptable en dérivant.

9. L'utilisation selon la revendication 4, dans laquelle le composé de formule II représente le 2-butyl-4-chloro-1-[(2'-carboxybiphényl-4-yl)méthyl]imidazole-5-carboxaldéhyde ou un sel pharmaceutiquement acceptable en dérivant.

10. L'utilisation selon la revendication 4, dans laquelle le composé de formule II représente le 2-butyl-1-[2'-carboxybiphényl-4-yl)méthyl]-imidazole-5-carboxaldéhyde ou un sel pharmaceutiquement acceptable en dérivant.

11. L'utilisation selon la revendication 4, dans laquelle le composé de formule II représente le 2-(1E-butényl)-4-chloro-1-[(2'carboxybiophényl-4-yl)méthyl]-5-(hydroxyméthyl)imidazole ou un sel pharmaceutiquement acceptable en dérivant.

12. L'utilisation selon la revendication 4, dans laquelle le composé de formule II représente le 2-(1E-butényl)-4-chloro-1-[(2'-carboxybiphényl-4-yl)méthyl]imidazole-5-carboxaldéhyde ou un sel pharmaceutiquement acceptable en dérivant.

13. L'utilisation selon la revendication 4, dans laquelle le composé de fromule II représente le 2-propyl-4-chloro-1-[(2'-(1H-tétrazol-5-yl)biphényl-4-yl)méthyl]imidazole-5-(hydroxyméthyl) imidazole ou un sel pharmaceutiquement acceptable en dérivant.

14. L'utilisation selon la revendication 4, dans laquelle le composé de formule II représente le 2-propyl-4-chloro-1-[(2'-(1H-tétrazol-5-yl)biphényl-4-yl)méthyl]imidazole-5-carboxaldéhyde ou un sel pharmaceutiquement acceptable en dérivant.

15. L'utilisation selon la revendication 4, dans laquelle le composé de formule II représente LE 2-butyl-4-chloro-1-[2'-(1H-tétrazol-5-yl)biphényl-4-yl)méthyl]imidazole-5-carboxaldéhyde ou un sel pharmaceutiquement acceptable en dérivant.

16. L'utilisation selon la revendication 4, dans laquelle le composé de formule II représente le 2-(1E-butényl)-4-chloro-1-[(2'-(1H-tétrazol-5-yl)biphényl-4-yl)méthyl)](hydroxyméthyl)imidazole ou un sel pharmaceutiquement acceptable en dérivant.

17. L'utilisation selon la revendication 4, dans laquelle le composé de formule II représente le 2-(1E-butényl)-4-chloro-1-[(2'-1H-tétrazol-5-yl)biphényl-4-yl)méthyl]imidazole-5-carboxaldéhyde ou un sel pharmaceutiquement acceptable en dérivant.

18. L'utilisation selon la revendication 4, dans laquelle le composé de formule II représente l'acide 2-butyl-4-chloro-1-[(2'-(1H-tétrazol-5-yl)biphényl-4-yl)méthyl]imidazole-5-carboxylique ou un sel pharmaceutiquement acceptable en dérivant.

19. L'utilisation selon la revendication 4, dans laquelle le composé de formule II représente l'acide 2-propyl-4-chloro-1-[(2'-(1H-tétrazol-5-yl)-biphényl-4-yl)méthyl]imidazole-5-carboxylique ou un sel pharmaceutiquement acceptable en dérivant.

20. L'utilisation selon la revendication 4, dans laquelle le composé de formule II représente l'acide 2-propyl-4-trifluorométhyl-1-[(2'-(1H-tétrazol-5-yl)biphényl-4-yl)méthyl]imidazole-5-carboxyxlique ou un sel pharmaceutiquement acceptable en dérivant.

21. L'utilisation selon la revendication 4, dans laquelle le composé de formule II représente le 2-propyl-4-trifluorométhyl-1-[(2'-(1H-tétrazol-5-yl)biphényl-4-yl)méthyl]-5-(hydyroxylméthyl)-imidazole ou un sel pharmaceutiquement acceptable en dérivant.

22. L'utilisation selon la revendication 4, dans laquelle le composé de formule II représente l'acide 2-butyl-4-trifluorométhyl-1-[(2'-(1H-tétrazol-5-yl)biphényl-4-yl)méthyl]imidazole-5-carboxylique ou un sel pharmaceutiquement acceptable en dérivant.

23. L'utilisation selon la revendication 4, dans laquelle le composé de formule II représente le 2-propyl-4-trifluorométhyl-1-[(2'-(carboxybiphényls-4-yl)méthyl]imidazole-5-carboxaldéhyde.

24. L'utilisation selon la revendication 4, dans laquelle le composé de formule 2 représente l'acide 2-propyl-4-pentafluoroéthyl-1-[(2'-(1H-tétrazol-5-yl)biphényl-4-yl)méthyl]-5-(hydroxyméthyl)-imidazole ou un sel pharmaceutiquement acceptable en dérivant.

25. L'utilisation selon la revendication 4, dans laquelle le composé de formule II représente l'acide 2-propyl-4-pentafluoroéthyl-1-[(2'-1H-tétrazol-5-yl)biphényl-4-yl)méthyl]imidazole-5-carboxylique ou un sel pharmaceutiquement acceptable en dérivant.

26. L'utilisation selon la revendication 4, dans laquelle le composé de formule II représente le 2-propyl-4-pentafluoroéthyl-[(2'-(1H-tétrazol-5-yl)biphényl-4-yl)méthyl]imidazole-5-carboxaldéhyde ou un sel pharmaceutiquement acceptable en dérivant.

27. L'utilisation selon la revendication 4, dans laquelle le composé de formule II représente le 1-[(2'-1H-tétrazol-5-yl)biphényl-4-yl)méthyl]-4-phényl-2-propylimidazole-5-carboxaldéhyde ou un sel pharmaceutiquement acceptable en dérivant.

28. L'utilisation selon la revendication 4, dans laquelle le composé de formule II représente le 1-[(2'-carboxybiphényl-4-yl)méthyl]-4-phényl-2-propylimidazole-5-carboxaldéhyde ou un sel pharmaceutiquement acceptable en dérivant.
